# EUROPEAN PATENT APPLICATION

(11) **EP 2 757 154 A1**
(43) Date of publication of application: **23.07.2014**
(21) Application number: 12832069.4
(22) Date of filing: 14.03.2012
(51) Int. Cl.: C12N 15/09, C12Q 1/68

(54) **METHOD FOR DETECTING BLADDER CANCER CELLS, PRIMER USED IN METHOD FOR DETECTING BLADDER CANCER CELLS, AND BLADDER CANCER MARKER**

(30) Priority: 16.09.2011 JP 2011203705
(71) Applicant: LSIP, LLC, Chiyoda-ku Tokyo 100-0005 (JP)
(72) Inventor: SHIMIZU, Takashi, Sapporo-shi Hokkaido 060-8556 (JP); SUZUKI, Hiromu, Sapporo-shi Hokkaido 060-8556 (JP); TOYOTA, Minoru, Deceased (JP); TSUKAMOTO, Taiji, Sapporo-shi Hokkaido 060-8556 (JP)
(74) Representative: Cabinet Plasseraud
(86) International application number: PCT/JP2012/056605
(87) International publication number: WO 2013/038737

(57) **Abstract**

Provided are: a method for detecting bladder cancer with high detection sensitivity, which has a high specificity for bladder cancer and is capable of detecting bladder cancer tissues with low grade of malignancy; and a bladder cancer marker. A method for detecting bladder cancer cells, which comprises detection of the amount of expressed bladder cancer marker in a subject sample collected from a subject, said bladder cancer marker being composed of one or more miRNAs selected from among miR-124, miR-9 and miR-137; a primer which is used in the method for detecting bladder cancer cells; and a bladder cancer marker.

## Description

### TECHNICAL FIELD

The present invention relates to a method for detecting cancer of a subject in a sample obtained from the subject, and specifically, to a method for detecting bladder cancer cells, a primer used in the method thereof, and a material that serves as a bladder cancer marker.

### BACKGROUND ART

Currently, urinary cytodiagnosis is most widely used for detecting bladder cancer. For urinary cytodiagnosis, cancer cells are detected by collecting bladder cells, which are released in urine after being detached from the bladder, from the urine, and then observing the form of the bladder cells with a microscope.

Meanwhile, research has been carried out on a method for performing the detection of cancer tissues by detecting the expression amount of microRNA (hereinafter, "microRNA" may be exchangeably used with each of "miRNA", "miR", and "hsa-miRNA") that is short RNA performing control of genes, for the tissues. As such a technique, Patent Document 1 discloses a method for detecting cancers in which canceration of a specimen is detected using a decrease in the expression of a miRNA gene including miR-9 and miR-137 as an indicator in the specimen, a method for suppressing cancers by expressing the miRNA, and a cancer inhibitor.

Patent Document 2 discloses a method for detecting whether or not breast cancers are present or a risk of developing breast cancers is present by measuring the levels of miRNA gene products in a test sample originating from a subject.

Patent Document 3 discloses, as a method for increasing the effectiveness of an anticancer treatment of BCL2-related cancers a method for diagnosing and treating BCL2-related cancers by administering at least one anticancer treatment to a subject and administering at least one miR gene product composed of the nucleotide sequence that is complementary to a nucleotide sequence in a BCL2 gene transcription body to a subject. To be specific, a validation result for the administration of miR-15 and miR-16 among miR genes in chronic lymphocytic leukemia among cancers is disclosed. The bladder is described as a cancer tissue having the possibility of decrease in the expression of miRNA. In addition, miR-137 and miR-9 are exemplified as miRNA capable of being used for diagnosing and treating BCL2-related cancers.

Patent Document 4 discloses a method for determining gynecology cancers using microRNA including miR-137 as a biomarker for gynecology cancers. As the method for determining gynecology cancers, a method is described for directly detecting the expression amount of miRNA.

Patent Document 5 discloses a method for predicting the survival of a cancer patient after being treated, and the method for predicting the survival of a cancer patient includes detecting the expression level of microRNA including hsa-miR137 of a cancer patient being treated, calculating the risk score of the patient on the basis of the expression level of the microRNA, and determining the survival prospect after the treatment on the basis of the risk score value. As examples of cancers to be predicted, lung cancer, leukemia, breast cancer, pancreatic cancer, adenocarcinoma, squamous cell carcinoma, colon cancer, or hepatocellular carcinoma are described.

Patent Document 6 discloses a method for determining any one of whether or not solid cancer is present and whether or not the risk of developing the solid cancer is present in a subject by measuring the level of at least one miR gene product.

Non-Patent Document 1 describes the expression of miR-137 in colorectal cancer. Non-Patent Document 2 describes the expression of miRNA including miR-137 in cavity cancer. Non-Patent Document 3 describes the expression of miR-137 in colorectal cancer.
[Patent Document 1] Japanese Unexamined Patent Application, Publication No. 2009-171876
[Patent Document 2] Japanese Unexamined Patent Application (Translation of PCT Application), Publication No. 2009-505639
[Patent Document 3] Japanese Unexamined Patent Application (Translation of PCT Application), Publication No. 2009-507918
[Patent Document 4] Japanese Unexamined Patent Application, Publication No. 2010-154843
[Patent Document 5] Japanese Unexamined Patent Application (Translation of PCT Application), Publication No. 2010-523156
[Patent Document 6] Japanese Unexamined Patent Application (Translation of PCT Application), Publication No. 2009-531019
[Non-Patent Document 1] F. BALAGUER ET AL, CANCER RES 2010;70:6609-6618.
[Non-Patent Document 2] K. KOZAKI ET AL, CANCER RES 2008;68:2094-2105.
[Non-Patent Document 3] M. LIU ET AL, INT. J. CANCER: 128, 1269-1279 (2011).

### DISCLOSURE OF THE INVENTION

### Problems to be Solved by the Invention

There has been a problem with sensitivity for detecting bladder cancer in urinary cytodiagnosis, which is currently widely used for detecting bladder cancer. In other words, for urinary cytodiagnosis, the shapes of cells are observed by using a microscope. Therefore, if the cells capable of being clearly confirmed as being tumor cells from the shapes of the cells can be detected, the bladder cancer cells can be specifically detected. However, the tumor cells with low malignancy, which cannot be confirmed from their shapes, cannot be detected. Therefore, it has been difficult to detect tumors with low malignancy.

Therefore, the present inventors focused on a method for genetically detecting cancer cells, especially, a method for detecting cancer by detecting miRNA, in order to obtain the specificity to cancer cells and the detection sensitivity capable of detecting a tumor with low malignancy. It is to be noted that Patent Document 1 describes a method for detecting cancers by detecting miRNA, a method for suppressing cancers by expressing the miRNA, and a cancer inhibitor. However, such disclosures are for oral squamous cell carcinoma, and are not validated regarding the tissues of bladder cancers.

Patent Document 2 describes a method and composition for detecting breast cancers or a risk of developing breast cancers, but such a method and composition are not validated regarding tissues of bladder cancers.

In Patent Document 3, the bladder is included as one among various exemplified tumor tissues in which the expression of miRNA may be generally decreased, but the validation is not performed specifically regarding the genes having the decreased expressions in bladder cancer and there is no evidence that the genes can be applied for bladder cancer. Examples of a miRNA gene to be administered include miR-137 and miR-9, but they are only exemplified as one among various candidates of miRNAs having the possibility of theoretically decreasing the expression of a BCL2 gene under the hypothesis, in which a decrease in the expression of the BCL2 gene leads to a cancer treatment. In addition, whether or not the expression of the BCL2 gene is actually decreased by such miRNA genes is not validated. In addition, this method is limited to the possibility of the treatment of the cancer related to excessive expression of the BCL2 gene and/or gene product, and whether or not the administration of miR-137 and miR-9 actually leads to the cancer treatment is not validated at all, either.

Patent Document 4 describes a method and kit for determining so-called gynecology cancers, but does not describe bladder cancer. In addition, on the contrary, an increase in the expression of miR-137 has been confirmed in cancer of the uterine body.

Patent Document 5 discloses a method for predicting the survival of a cancer patient after being treated, but does not describe a method for detecting whether or not a patient has cancer. In addition, bladder cancer is not described as a cancer.

In Patent Document 6, the expression of miR-137 does not change in six kinds of cancers (breast, large intestine, lung, pancreas, prostate, and stomach). The genes, which are not selected as a gene to be measured, are exemplified as miR-137, and especially, the genes which are not selected for measuring each of six kinds of cancer as described above are exemplified as miR-137.

Non-Patent Documents 1 to 3 describe the expression of miRNA including miR-137, the outbreak of colorectal cancer or oral cancer, and the relation with permeation of colorectal cancer, respectively, but these are not validated regarding bladder cancer.

As described above, there are various differences in related miRNA or expression level thereof according to the type of cancer or the state of cancer, and a complicated mechanism is indicated. Therefore, the present inventors searched miRNA having the suppressed expression in bladder cancer. As a result, the present inventors found the miRNA having the suppressed expression in bladder cancer, and measured the expression amount thereof, thereby finding a method for detecting the bladder cancer. Therefore, the present inventors completed the present invention.

Therefore, an object of the present invention is to provide a method for detecting bladder cancer cells, primers used in the method, and a bladder cancer marker, in which the method has high specificity to bladder cancer and high detection sensitivity capable of detecting bladder cancer tissues with low malignancy.

### Means for Solving the Problems

The method for detecting bladder cancer cells according to the present invention includes detecting an expression amount of a bladder cancer marker including one or more miRNAs selected from miR-124, miR-9, and miR-137 in a subject sample collected from a subject.

In bladder cancer tissues, the miRNA having the expression amount specifically different as compared with normal tissues is detected as the bladder cancer marker. Since for the miRNA in tissues, whether or not the expression thereof is generated can be quickly and accurately detected and the expression amount thereof can be quantitatively detected using a means, such as a real time reverse-transcription PCR (a real time reverse transcription PCR, a real time RT-PCR, and a quantitative RT-PCR), the bladder cancer cells can be specifically detected by detecting the expression amount of one or more miRNAs selected from miR-124, miR-9, and miR-137, and even the bladder cancer tissues with low malignancy can be detected.

The detection of the expression amount of the bladder cancer marker is preferably to detect a decrease in the expression amount of the bladder cancer marker by detecting the methylation (methylated cytosine) of the gene of the bladder cancer marker. Since in the bladder cancer cells, the expressions of miR-124, miR-9, miR-137, and the like are suppressed by the methylation on a genome gene encoding each of them, decreases in the expression amounts of these miRNAs in the bladder cancer cells and the bladder cancer cells can be detected by detecting the methylation of the miR-124-2 gene and the miR-124-3 gene for the miR-124, the miR-9-3 gene for the miR-9, and the miR-137 gene for the miR-137. In a case in which cancer tissues are included in the normal tissues expressing target miRNA in a large quantity, since the expression in the normal tissues is detected using a means for directly detecting the expression amount, it is difficult to detect the target miRNA in cancer tissues in some cases. However, the existence thereof can be clearly and reliably detected by detecting the decrease in the expression amount as a positive signal that is the methylation.

It is to be noted that a method for detecting bladder cancer cells includes detecting the levels of the methylation of one or more genes selected from the miR-137 gene, the miR-124-2 gene, the miR-124-3 gene, and the miR-9-3 gene in a subject sample collected from a subject. In this detection method, it is preferable to compare the level of methylation with a threshold value. The levels of the methylation of the above-described genes in different tissues that are identified as non-cancerous tissues, tissues of other sites, or the like are defined as threshold values, and then the levels of the methylation of the above-described genes in the tissues to be detected are compared with the threshold values, and thereby the bladder cancer cells can be detected. In a case in which the level of the methylation in the subject sample is high as compared with the threshold value, the subject sample can be determined to be a cancer tissue.

The detection of the methylation is preferably performed by a bisulfite pyrosequencing method. With this method, the detection of targeted miRNA can be accurately and quantitatively performed. Therefore, the detection of the tissues of the bladder cancer cells can be reliably performed. The bladder cancer marker preferably includes at least the miR-137. In bladder cancer cells, the expression amount of miR-137 exhibits a particularly remarkable difference as compared with normal tissue, and thus the bladder cancer cells can be detected with the miR-137 to a higher degree of reliability.

It is preferable to compare the expression amount of the bladder cancer marker in the subject sample with a threshold value. The expression amounts of miRNA in different tissues that are identified as a non-cancerous tissue, tissues of other sites, or the like are defined as threshold values, and then the expression amount of the bladder cancer marker is compared with the threshold values, and thereby the cancer tissues can be detected. In a case in which the expression amount of the bladder cancer marker in the subject sample is low as compared with the threshold value, the subject sample can be determined to be a cancer tissue.

The threshold value is preferably the expression amount of the bladder cancer marker in a control sample collected from normal tissues. Since the expressions of miR-124, miR-9, and miR-137 are suppressed in bladder cancer cells, the bladder cancer cells can be detected by the decrease in the expression amount as compared with the normal tissues.

The threshold value is preferably the expression amount of the bladder cancer marker in a control sample collected from the subject at other times or collected from other tissues of the subject. The bladder cancer cells can be detected through the comparison with the time when cancer was not developed or at the time when cancer was excised by comparing with the control sample collected from the subject at other times. Since temporal data relating to the expression of the bladder cancer cells can be obtained, cancer occurrence or cancer treatment outcome can be determined. The bladder cancer cells can be detected through the comparison with the tissues without cancer by comparing with the control sample that is collected from other tissues of the subject. The sites of tissues in which cancer is developed can be detected by collecting a sample from each of the sites.

The subject sample is preferably a urine sample. A urine sample can be safely, quickly, simply and frequently collected without using surgery and without invasiveness. Uroepithelial cells that are detached and released in urine are detected in a urine sample, and thus the amount of miRNA contained is small as compared with a blood sample or a sample collected through excision. However, in the present embodiment, the detection of the methylation is performed by a bisulfite pyrosequencing method, and thus it is possible to perform detection in the urine sample with sufficiently high specificity and high sensitivity.

In the detection of the expression amount by detecting the methylation of the bladder cancer marker, as for a primer used in the method for detecting bladder cancer cells, the sequences of primers used for amplifying the miR-137 gene by a bisulfite pyrosequencing method are preferably a forward primer (GGGTTTAGYGAGTAGTAAGAGTTTTG) represented by SEQ ID NO 1 and a reverse primer (CCCCCTACCRCTAATACTCTCCTC) represented by SEQ ID NO 2, and the sequence of the primer used for the sequencing reaction is preferably GGTATTTTTGGGTGGATAAT represented by SEQ ID NO 3.

In the detection of the expression amount by detecting the methylation of the bladder cancer marker, as for the primer used in the method for detecting bladder cancer cells, the sequences of primers used for amplifying the miR-124-2 gene by a bisulfide pyrosequencing method are preferably a forward primer (GTTGGGATTGTATAGAAGGATTATTTG) represented by SEQ ID NO 4 and a reverse primer (ACTACRAAAATCCAAAAAAAAATACATAC) represented by SEQ ID NO 5, and the sequence of the primer used for the sequencing reaction is preferably YGTTTTTATTGTTTTAGTTT represented by SEQ ID NO 6.

In the detection of the expression amount by detecting the methylation of the bladder cancer marker, as for the primer used in the method for detecting bladder cancer cells, the sequences of primers used for amplifying the miR-124-3 gene by a bisulfite pyrosequencing method are preferably a forward primer (AAAAGAGAYGAGTTTTTATTTTTGAGTAT) represented by SEQ ID NO 7 and a reverse primer (TCCTCCRCAACTACCTTCCCCTA) represented by SEQ ID NO 8, and the sequence of the primer used for the sequencing reaction is preferably GAGATTYGTTTTTTTAAT represented by SEQ ID NO 9.

In the detection of the expression amount by detecting the methylation of the bladder cancer marker, as for the primer used in the method for detecting bladder cancer cells, the sequences of primers used for amplifying the miR-9-3 gene by a bisulfite pyrosequencing method are preferably a forward primer (GATTTGAATGGGAGTTTGTGATTGGT) represented by SEQ ID NO 10 and a reverse primer (TCCCRAAACTCACRTAAAACACCC) represented by SEQ ID NO 11, and the sequence of the primer used for the sequencing reaction is preferably TTGGATTGAYGTTATTTT represented by SEQ ID NO 12.

It is also preferable to perform the detection of the methylation by a methylation-specific PCR method (MSP method). According to the method, the detection of the methylation of a targeted miRNA gene can be performed quickly and simply in a small amount of a DNA sample, and thus the detection of the tissues of the bladder cancer can be reliably performed.

In the methylation-specific PCR method, as for a primer used in the method for detecting bladder cancer cells, the sequences of primers used for detecting the methylation of the miR-137 gene are preferably a forward primer (GTAGCGGTAGTAGCGGTAGCGGT) represented by SEQ ID NO 13 and a reverse primer (GCTAATACTCTCCTCGACTACGCG) represented by SEQ ID NO 14 as allele-specific methylation primers and a forward primer (TGGTAGTGGTAGTAGTGGTAGTGGT) represented by SEQ ID NO 15 and a reverse primer (CCACTAATACTCTCCTCAACTACACA) represented by SEQ ID NO 16 as unmethylated allele-specific primers.

In the methylation-specific PCR method, as for a primer used in the method for detecting bladder cancer cells, the sequences of primers used for detecting the methylation of the miR-124-2 gene are preferably a forward primer (AGGGGCGTATTTTGGGGTTTTTGC) represented by SEQ ID NO 17 and a reverse primer (CCCCTACGACGTAATCGACCCG) represented by SEQ ID NO 18 as allele-specific methylation primers and a forward primer (TTTAGGGGTGTATTTTGGGGTTTTTGT) represented by SEQ ID NO 19 and a reverse primer (CATCCCCTACAACATAATCAACCCA) represented by SEQ ID NO 20 as unmethylated allele-specific primers.

In the methylation-specific PCR method, as for a primer used in the method for detecting bladder cancer cells, the sequences of primers used for detecting the methylation of the miR-124-3 gene are preferably a forward primer (GTTTTAGTGATAATCGGTCGGTGTC) represented by SEQ ID NO 21 and a reverse primer (TCCACGAAATCCACGCTACAAACG) represented by SEQ ID NO 22 as allele-specific methylation primers and a forward primer (TGTGTTTTAGTGATAATTGGTTGGTGTT) represented by SEQ ID NO 23 and a reverse primer (ATATCCACAAAATCCACACTACAAACA) represented by SEQ ID NO 24 as unmethylated allele-specific primers.

In the methylation-specific PCR method, as for a primer used in the method for detecting bladder cancer cells, the sequences of the primers used for detecting the methylation of the miR-9-3 gene are preferably a forward primer (GATTGACGTTATTTTTTCGCGGGGC) represented by SEQ ID NO 25 and a reverse primer (CGAAACTCACGTAAAACACCCGCG) represented by SEQ ID NO 26 as allele-specific methylation primers and a forward primer (TTGGATTGATGTTATTTTTTTGTGGGGT) represented by SEQ ID NO 27 and a reverse primer (CCCAAAACTCACATAAAACACCCACA) represented by SEQ ID NO 28 as unmethylated allele-specific primers.

A bladder cancer marker of the present invention contains one or more miRNAs selected from miR-124, miR-9, and miR-137.

In the tissues of bladder cancer, the miRNA that has a specifically different expression amount as compared with the normal tissues is detected as the bladder cancer marker by detecting the expression amount of the bladder cancer marker in a subject sample collected from a subject. For the expression amount of miRNA or the level of the methylation of the corresponding gene in a tissue, whether or not the expression thereof is generated can be quickly and accurately detected and the amount thereof can be quantitatively detected using a means such as a real time PCR or a bisulfite pyrosequencing method, and thus bladder cancer cells can be specifically detected by detecting the expression amounts of one or more miRNAs selected from miR-124, miR-9, and miR-137 or the levels of the methylation of the corresponding genes, and also even the bladder cancer tissues with low malignancy can be detected.

The bladder cancer marker of the present invention is used in a method for detecting bladder cancer cells, in which the method includes detecting the expression amount of the bladder cancer marker in a subject sample collected from a subject. The detection of the expression amount of the bladder cancer marker is used in the method for detecting bladder cancer cells, in which the method includes detecting the decrease in the expression amount of the bladder cancer marker by the detection of the methylation of a genome gene encoding the bladder cancer marker.

In the method for detecting bladder cancer cells of the present invention, the detection is performed in a subject sample collected from a subject exhibiting pTa of an invasion depth or G1/G2 of an atypical degree. The detection of primary cancer can be effectively performed by detecting the level of the methylation of a miRNA gene for a patient with the primary cancer.

A nucleic acid molecule of the present invention has a nucleotide sequence of SEQ ID NO 1, SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24, SEQ ID NO 25, SEQ ID NO 26, SEQ ID NO 27, or SEQ ID NO 28. These molecules can be used in the method for detecting bladder cancer cells.

### Effects of the Invention

According to the present invention, in the tissues of bladder cancer, the miRNA that has a specifically different expression amount as compared with normal tissues is detected as the bladder cancer marker. The expression amount of miRNA or the level of the methylation of the corresponding gene in a tissue can be quickly, accurately, and quantitatively detected by a means such as a real time PCR or a bisulfite pyrosequencing method. Therefore, by detecting the expression amounts of one or more miRNAs selected from miR-124, miR-9, and miR-137, and the levels of the methylation of the corresponding genes, bladder cancer cells can be specifically detected and also even bladder cancer tissues with low malignancy can be detected.

### BRIEF DESCRIPTION OF THE DRAWINGS

Fig. 1 is a diagram illustrating results obtained by analyzing the state of the methylation for each of the genome gene sequences encoding miRNAs using bladder cancer cell lines by a methylation-specific PCR method.
Fig. 2 is graphs illustrating the results obtained by analyzing the state of the methylation for miR-9-1, miR-9-3, miR-10b, and miR-34b among miRNAs of Fig. 1 by a bisulfite pyrosequencing method.
Fig. 3 is graphs illustrating the results obtained by analyzing the state of the methylation for miR-124-1, miR-124-2, miR-124-3, and miR-137 among miRNAs of Fig. 1 by a bisulfite pyrosequencing method.
Fig. 4 is graphs illustrating the results obtained by analyzing the state of the methylation for miR-200b, miR-203, miR-409, and miR-675 among miRNAs of Fig. 1 by a bisulfite pyrosequencing method.
Figs. 5(a) and 5(b) are graphs illustrating the results obtained by analyzing (a) the expression amount of the miRNA of miR-137 and (b) the methylation of the miR-137 gene for each of the bladder cancer cell lines.
Fig. 6 is a graph illustrating the results obtained by analyzing the expression of miR-137 in a cancer part tissue (T) and a tissue (DN) that is considered to be normal.
Figs. 7(a) and 7(b) are graphs illustrating (a) the results obtained by analyzing the expression amount of miR-137 and (b) the results obtained by analyzing the methylation in a cancer part tissue (T) and a tissue (DN) that is considered to be normal.
Figs. 8(a) and 8(b) are graphs illustrating the results obtained by analyzing the methylation of miR-137 in tissues collected from each of a cancer part tissue (T), a tissue (DN) that is considered to be normal, and a tissue (AN; Adjacent Normal-appearing bladder tissue) that is 5 mm apart from the cancer part (collected tissue of T) of cases of (a) NMIBC (non-invasiveness and superficial property) and (b) MIBC (invasiveness), by a bisulfite pyrosequencing method.
Fig. 9 is graphs illustrating the results obtained by analyzing the methylation of a case of NMIBC (non-invasiveness and superficial property), a case of MIBC (invasiveness), and both cases for the miR-137 gene by a bisulfite pyrosequencing method, and the results obtained by analyzing the ROC curves thereof.
Fig. 10 is graphs illustrating the results obtained by analyzing the methylation of a case of NMIBC (non-invasiveness and superficial property), a case of MIBC (invasiveness) and both cases for the miR-124-2 gene by a bisulfite pyrosequencing method, and the results obtained by analyzing the ROC curves thereof.
Fig. 11 is graphs illustrating the results obtained by analyzing the methylation of a case of NMIBC (non-invasiveness and superficial property), a case of MIBC (invasiveness) and both cases for the miR-124-3 gene by a bisulfite pyrosequencing method, and the results obtained by analyzing the ROC curves thereof.
Fig. 12 is graphs illustrating the results obtained by analyzing the methylation of a case of NMIBC (non-invasiveness and superficial property), a case of MIBC (invasiveness), and both cases for the miR-9-3 gene by a bisulfite pyrosequencing method, and the results obtained by analyzing the ROC curves thereof.
Figs. 13(a) and 13(b) are graphs illustrating the results obtained by analyzing (a) the methylation of miR-137 genes in urine specimens before an operation for the removal of cancer tissues and after the removal of cancer tissues, and (b) the ROC curves thereof.
Figs. 14(a) and 14(b) are graphs illustrating the results obtained by analyzing the methylation of miR-137 genes in urine specimens (a) before and after an operation for the removal of cancer tissues and (b) before and after an operation on a non-cancerous patient.
Figs. 15(a) and 15(b) are graphs illustrating the results obtained by analyzing (a) the methylation of miR-124-2 genes of urine specimens before an operation for the removal of cancer tissues and after the removal of cancer tissues, and (b) the ROC curves thereof.
Figs. 16(a) and 16(b) are graphs illustrating the results obtained by analyzing the methylation of miR-124-2 genes in urine specimens (a) before and after an operation for the removal of cancer tissues and (b) before and after an operation on a non-cancerous patient.
Figs. 17(a) and 17(b) are graphs illustrating the results obtained by analyzing (a) the methylation of miR-124-3 genes of urine specimens before an operation for the removal of cancer tissues and after the removal of cancer tissues, and (b) the ROC curves thereof.
Figs. 18(a) and 18(b) are graphs illustrating the results obtained by analyzing the methylation of miR-124-3 genes in urine specimens (a) before and after an operation for the removal of cancer tissues and (b) before and after an operation on a non-cancerous patient.
Figs. 19(a) and 19(b) are graphs illustrating the results obtained by analyzing (a) the methylation of miR-9-3 genes of urine specimens before an operation for the removal of cancer tissues and after the removal of cancer tissues, and (b) the ROC curves thereof.
Figs. 20(a) and 20(b) are graphs illustrating the results obtained by analyzing the methylation of miR-9-3 genes in urine specimens (a) before and after an operation for the removal of cancer tissues and (b) before and after an operation on a non-cancerous patient.
Fig. 21 is schematic diagrams illustrating the training set of a panel detection method.
Fig. 22 is schematic diagrams illustrating the test set of a panel detection method.
Fig. 23 is schematic diagrams illustrating the training set of a detection method by a Tree diagram.
Fig. 24 is schematic diagrams illustrating the test set of a detection method by a Tree diagram.
Fig. 25 is a graph illustrating the results obtained by analyzing the suppressing effect on bladder cancer by forcibly expressing miR-137 in bladder cancer cell lines.
Fig. 26 is diagrams illustrating the sequences of the miR-137 gene and genome DNA near the miR-137 gene, and the sequences of primers used in the bisulfite pyrosequencing method and the methylation-specific PCR (MSP) method of the present embodiment.
Fig. 27 is diagrams illustrating the sequences of the miR-124-2 gene and genome DNA near the miR-124-2 gene, and the sequences of primers used in the bisulfite pyrosequencing method and the methylation-specific PCR (MSP) method of the present embodiment.
Fig. 28 is diagrams illustrating the sequences of the miR-124-3 gene and genome DNA near the miR-124-3 gene, and the sequences of primers used in the bisulfite pyrosequencing method and the methylation-specific PCR (MSP) method of the present embodiment.
Fig. 29 is diagrams illustrating the sequences of the miR-9-3 gene and genome DNA near the miR-9-3 gene, and the sequences of primers used in the bisulfite pyrosequencing method and the methylation-specific PCR (MSP) method of the present embodiment.

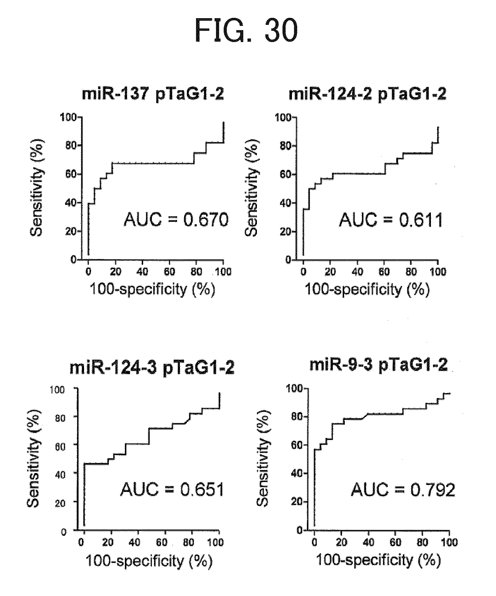

Fig. 30 is diagrams illustrating the results obtained by analyzing the methylation of miR-137, miR-124-2, miR-124-3, and miR-9-3 genes for a patient exhibiting pTa and G1/G2.
Fig. 31 is diagrams illustrating a panel detection method and a detection method by a Tree diagram on the basis of the results of Fig. 30.

### PREFERRED MODE FOR CARRYING OUT THE INVENTION

Hereinafter, the present invention will be described in detail with reference to embodiments.

### (Method for detecting bladder cancer cells)

The bladder cancer in the present embodiment refers to cancer such as urothelial cancer (transitional cell carcinoma), squamous cell carcinoma, or adenocarcinoma, which develops in the bladder.

The present embodiment uses one or more miRNAs selected from at least one of miR-124, miR-9, and miR-137 as a bladder cancer marker. The miRNA has the structure of 5'-phosphoric acid and 3'-OH in chain RNA having one short chain of 10 or more base pairs having the sequences to be described below, in which two bases are projected on the 3'-end in some cases. In the present embodiment, the miRNA refers to all of the miRNAs that are artificially chemo-synthesized and the miRNAs that are synthesized in a living organism.

The information of the sequences of miRNAs and the sequences of genome genes encoding the respective miRNAs used in the present invention are registered in the miRBASE database (http://microrna.sanger.ac.uk/). The respective sequences and Accession Nos are as follows.
miR-137 (5'-uuauugcuuaagaauacgcguag-3') (MIMAT0000429) (SEQ ID NO 29)
miR-124 (5'-uaaggcacgcggugaaugcc-3') (MIMAT0000422) (SEQ ID NO 30)
miR-9 (5'-ucuuugguuaucuagcuguauga-3') (MIMAT0000441) (SEQ ID NO 31)
miR-137 gene (5'-ggtcctctgactctcttcggtgacgggtattcttgggtggataatacggattacgttgttat tgcttaagaatacgcgtagtcgaggagagtaccagcggca-3') (MI0000454) (SEQ ID NO 32)
miR-124-2 gene (5'-atcaagattagaggctctgctctccgtgttcacagcggaccttgatttaatgtcatacaatt aaggcacgcggtgaatgccaagagcggagcctacggctgcacttgaa-3') (MI0000444) (SEQ ID NO 33)
miR-124-3 gene (5'-tgagggcccctctgcgtgttcacagcggaccttgatttaatgtctatacaattaaggcacgc ggtgaatgccaagagaggcgcctcc-3') (MI0000445) (SEQ ID NO 34)
miR-9-3 gene (5'-ggaggcccgtttctctctttggttatctagctgtatgagtgccacagagccgtcataaagct agataaccgaaagtagaaatgattctca-3') (MI0000468) (SEQ ID NO 35)

The present inventors have found that it is possible to use miR-137 among those described above as a clear indicator of bladder cancer cells of bladder cancer. In addition, a plurality of miRNAs among the above-described miRNAs may be detected, and then the results thus obtained may be compared and validated. By comparing and validating the plurality of miRNAs, the detection can be performed with higher specificity and sensitivity.

As a method for detecting the expression amount of a bladder cancer marker, that is, for detecting the above-described miRNAs in the present embodiment, a method for detecting the expression amount of the miRNAs in a subject sample collected from a subject, which is a target for detecting bladder cancer cells are detected, can be adapted. As the subject sample, bladder cancer tissues or living body samples including the tissues may be collected, and then used. The detection may be performed in a urine sample, a blood sample, a sample that is collected after being excised through an endoscope, or the like. In the present embodiments, a sample that is collected after being excised through an endoscope or samples that are collected from all bladder specimens are used.

The detection of the expression amount of an intended bladder cancer marker is performed by comparing the expression amount of the bladder cancer marker in a subject sample collected from a subject that is a target for detecting bladder cancer cells with a threshold value. In a case in which the expression amount of the bladder cancer marker in the subject sample decreases as compared with the threshold value, the tissues of the subject sample can be determined to be bladder cancer. The threshold value is obtained from a control sample collected from normal tissues, and then compared with the expression amount. This control sample may be from a normal tissue of a normal subject different from the subjects with suspected bladder cancer, or may be from the tissues of the same subject which are collected when the subject with suspected bladder cancer has normal health or from the healthy tissues of the subject with suspected bladder cancer. In the present embodiment, a normal tissue that is sufficiently apart from the tissues that may have cancer in the same subject is used as a control sample.

The detection of the expression amount of the bladder cancer marker described above in the bladder cancer cells is preferably performed by detecting and comparing the methylation of genome sequences encoding the intended miRNAs. In a case in which the frequency of the methylation of the genome sequence encoding the intended miRNA in the subject sample is higher as compared with the control sample, the expression amount of the intended miRNA decreases. For this reason, when the frequency of the methylation of the genome sequence encoding the intended miRNA in the subject sample is high, the existence of bladder cancer cells can be determined. By the detection of the methylation, high precision detection can be performed regardless of the background of the expression amount of the miRNA in the tissues. The detection of the methylation may use various methylation detection means, for example, a bisulfite sequencing method, a methylation-specific PCR (MSP) method, a quantitative MSP method, a COBRA method, a bisulfite pyrosequencing method, and the like. Any of these methods may be used either alone or as a combination of two or more. Among these, the bisulfite pyrosequencing method is preferably used from the viewpoint that the detection of the targeted miRNA can be performed accurately and quantitatively, and the methylation-specific PCR (MSP) method is preferably used from the viewpoint that the detection of the methylation can be performed quickly and simply in a small amount of the DNA sample.

It is to be noted that the MSP method may be used by using or properly changing the methods that are disclosed in Methods Mol Med. 2005; 113:279-91, and Taku Suzuki, Minoru Toyota, Kozo Imai: bisulfite PCR method, New Genetic Engineering Handbook Revision Fourth Edition (edited by Masami Muramatsu, Tadashi Yamamoto), Yodosha, PP99-106, 2003; the bisulfite sequencing method may be used by using or properly changing the methods that are disclosed in Methods. 2002 Jun;27(2):101-7, and Taku Suzuki, Minoru Toyota, Kozo Imai: bisulfite PCR method, New Genetic Engineering Handbook Revision Fourth Edition (edited by Masami Muramatsu, Tadashi Yamamoto), Yodosha, pp99-106, 2003; the bisulfite pyrosequencing method may be used by using or properly changing the methods that are disclosed in Nat Protoc. 2007;2(9):2265-75; the COBRA method may be used by using or properly changing the methods that are disclosed in Methods Mol Biol. 2002;200:71-85, and Taku Suzuki, Minoru Toyota, Kozo Imai: bisulfite PCR method, New Genetic Engineering Handbook Revision Fourth Edition (edited by Masami Muramatsu, Tadashi Yamamoto), Yodosha, pp99-106, 2003; and a methylight method may be used by using or properly changing the methods that are disclosed in Methods. 2001 Dec; 25(4):456-62. It is to be noted that the bisulfite pyrosequencing method is generally abbreviated to a pyrosequencing method in some cases, and in this case, the pyrosequencing method is the same technique as the bisulfite pyrosequencing method in the present specification.

The detection of the expression amount of a gene may be performed by directly detecting the expression amount of the miRNA. In order to directly detect the expression amount of the miRNA, the detection means for the miRNA such as a real time RT-PCR method and a northern blotting method may be properly used. Any of these methods may be used either alone or as a combination of two or more. Among these, the detection by the real time RT-PCR method is preferably used from the viewpoint of simplicity and sensitivity.

The detection of bladder cancer cells may be performed by using either one of the direct detection of the expression amount of the intended miRNA and the detection of the methylation of a genome sequence encoding the intended miRNA, or by using both of them.

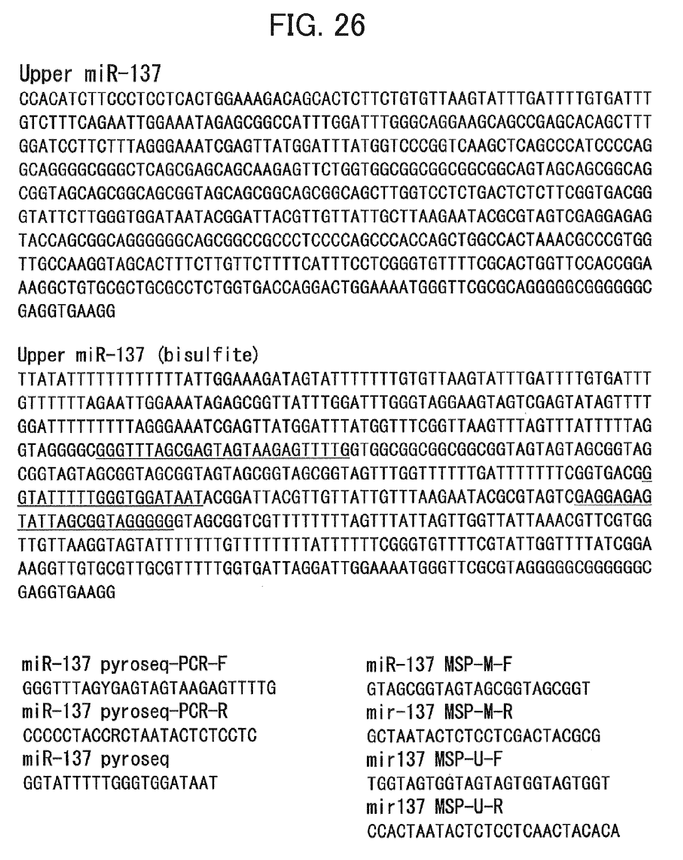

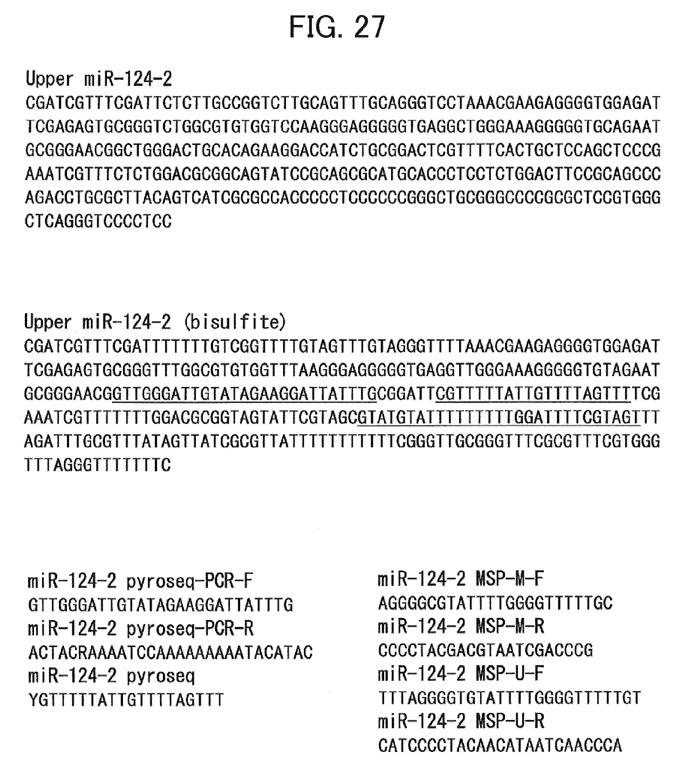

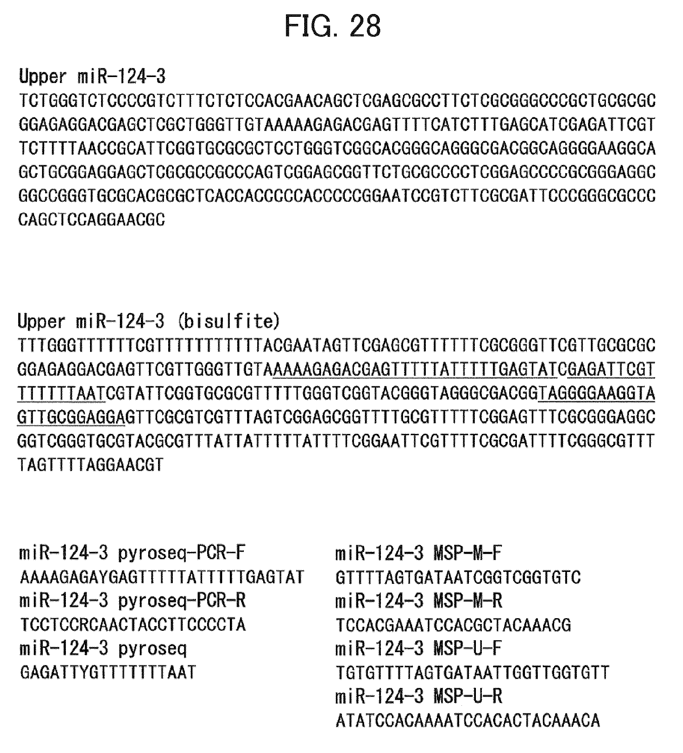

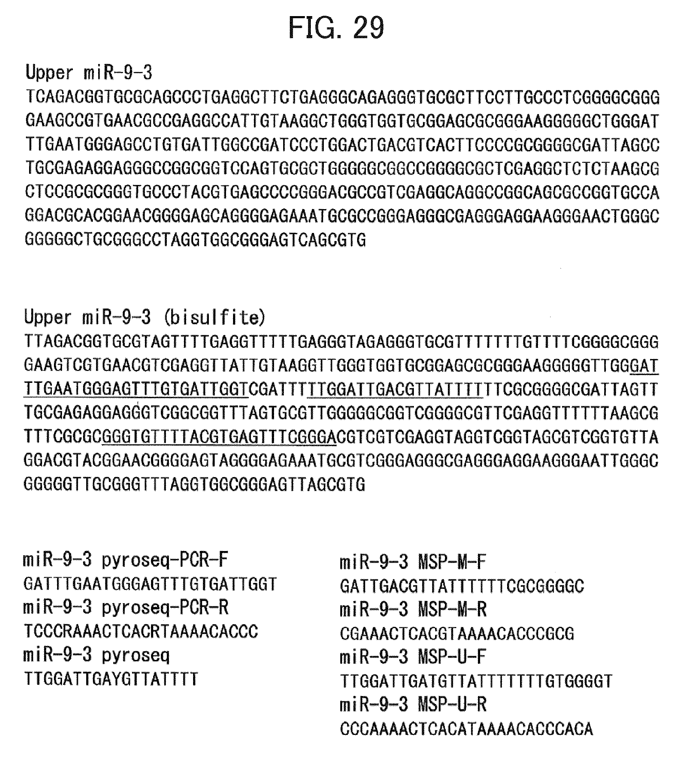

For the miR-137 gene, the miR-124-2 gene, the miR-124-3 gene, and the miR-9-3 gene, the primers used for detecting the methylation by a bisulfite pyrosequencing method, the primers used for detecting the methylation by an MSP method, and the sequences that are starting points thereof are illustrated in Figs. 26, 27, 28, and 29, respectively. The figures illustrate, for all of the miR-137 gene, the miR-124-2 gene, the miR-124-3 gene, and the miR-9-3 gene, the upper sequences (SEQ ID NOS 36, 38, 40 and 42), the sequences after a bisulfite conversion (the sequences used for primers represented at the underline part) (SEQ ID NOS 37, 39, 41, and 43), the forward primers and reverse primers used for a PCR when methylation allele and unmethylation allele are amplified at the same time, the primers used for a sequencing reaction after PCR amplification, and an example of the combinations of methylation allele-specific forward primers and reverse primers and the unmethylation allele-specific forward primers and reverse primers used for detecting by an MSP method.

For the miR-137 gene, a forward primer GGGTTTAGYGAGTAGTAAGAGTTTTG and a reverse primer CCCCCTACCRCTAATACTCTCCTC are used as primers used for PCR amplification by a bisulfite pyrosequencing method. GGTATTTTTGGGTGGATAAT is used as a primer used for a sequencing reaction.

For the miR-124-2 gene, a forward primer GTTGGGATTGTATAGAAGGATTATTTG and a reverse primer ACTACRAAAATCCAAAAAAAAATACATAC are used as primers used for PCR amplification by a bisulfite pyrosequencing method. YGTTTTTATTGTTTTAGTTT is used as a primer used for a sequencing reaction.

For the miR-124-3 gene, a forward primer AAAAGAGAYGAGTTTTTATTTTTGAGTAT and a reverse primer TCCTCCRCAACTACCTTCCCCTA are used as primers used for PCR amplification by a bisulfite pyrosequencing method. GAGATTYGTTTTTTTAAT is used as a primer used for a sequencing reaction.

For the miR-9-3 gene, a forward primer GATTTGAATGGGAGTTTGTGATTGGT and a reverse primer TCCCRAAACTCACRTAAAACACCC are used as primers used for PCR amplification by a bisulfite pyrosequencing method. TTGGATTGAYGTTATTTT is used as a primer used for a sequencing reaction.

The methylation-specific PCR method (MSP method) uses the following primers. For the sequences of primers used for detecting miR-137, a forward primer GTAGCGGTAGTAGCGGTAGCGGT and a reverse primer GCTAATACTCTCCTCGACTACGCG are used as allele-specific methylation primers and a forward primer TGGTAGTGGTAGTAGTGGTAGTGGT and a reverse primer CCACTAATACTCTCCTCAACTACACA are used as unmethylated allele-specific primers.

For the sequences of primers used for detecting miR-124-2, a forward primer AGGGGCGTATTTTGGGGTTTTTGC and a reverse primer CCCCTACGACGTAATCGACCCG are used as allele-specific methylation primers and a forward primer TTTAGGGGTGTATTTTGGGGTTTTTGT and a reverse primer CATCCCCTACAACATAATCAACCCA are used as unmethylated allele-specific primers.

For the sequences of primers used for detecting miR-124-3, a forward primer GTTTTAGTGATAATCGGTCGGTGTC and a reverse primer TCCACGAAATCCACGCTACAAACG are used as allele-specific methylation primers and a forward primer TGTGTTTTAGTGATAATTGGTTGGTGTT and a reverse primer ATATCCACAAAATCCACACTACAAACA are used as unmethylated allele-specific primers.

For the sequences of primers used for detecting miR-9-3, a forward primer GATTGACGTTATTTTTTCGCGGGGC and a reverse primer CGAAACTCACGTAAAACACCCGCG are used as allele-specific methylation primers and a forward primer TTGGATTGATGTTATTTTTTTGTGGGGT and a reverse primer CCCAAAACTCACATAAAACACCCACA are used as unmethylated allele-specific primers.

As a modified embodiment of the present embodiment, other sequences selected from the sequences after a bisulfite conversion as illustrated in Figs. 26, 27, 28, and 29 may be selected as primers of the miR-137 gene, the miR-124-2 gene, the miR-124-3 gene, and the miR-9-3 gene.

### (Diagnosing method using urine sample as subject sample)

In another embodiment of the present invention, the subject sample is a urine sample collected from a subject. As a control sample, a urine sample after performing the treatment of bladder cancer, for example, an operation for the excision of the bladder is used. The detection of the expression amount of the miRNA is performed by detecting the methylation like in the embodiment as described above. Other respects are similar to the above-described embodiment.

In the case in which the frequency of the methylation in the control sample is low as compared with the subject sample, it can be determined that bladder cancer tissues have been removed by the treatment.

In this embodiment, collecting a urine sample is not invasive for a subject, and thus the pain due to the collection is the minimum. In addition, the urine sample is very easily collected frequently, and thus is preferable. Uroepithelial cells that are detached and released in urine are detected in a urine sample, and thus the amount of the miRNA contained in the urine sample is small as compared with the sample collected through excision. However, in the present embodiment, the detection of the methylation is performed by a bisulfite pyrosequencing method, and thus it is possible to perform detection in the urine sample with sufficiently high specificity and sensitivity.

### (Bladder cancer inhibitor)

Another embodiment of the present invention is a bladder cancer inhibitor containing one or more miRNAs selected from miR-124, miR-9, and miR-137. The present inventors have found that bladder cancer can be suppressed by administering the genes having the suppressed expressions in cancer cells of bladder cancer, to cancer tissues, or expressing the genes in cancer tissues.

The bladder cancer inhibitor is, for example, a gene medicine, having the above-described miRNA as an effective component, in which such an effective component is combined with a base compound used for a gene medicine. For example, the bladder cancer inhibitor may be prepared as an injection that is solutionized or powdered after adding a buffer, amino acids, or other nutrients. Alternatively, the bladder cancer inhibitor may be properly combined with a base compound as needed in order to be prepared as a liquid drug or a sustained release agent.

The bladder cancer inhibitor may be prepared as a drug that introduces the above-described miRNA into a cell to express the miRNA in a cancer cell. For example, the bladder cancer inhibitor may be prepared as a drug for introducing nucleic acid molecules into a tissue by subsuming the above-described miRNA into a ribosome, and the like, a drug for the method of introducing nucleic acid molecules into a cell by a microinjection method, and the like, and a drug for introducing the above-described miRNA into a cell by administering it to a living body through a virus vector.

### EXAMPLES

### (Analysis of miRNA expression in cancer cell lines)

Bladder cancer cell lines (T24 and UM-UC-3) were treated with a DNA methylation enzyme inhibitor, 5-aza-2'-deoxycytidine (5-aza-dC), and an HDAC inhibitor, 4-phenylbutyric acid (4-PBA). Each of the expression profiles of the sample after being treated and the control without being treated was analyzed by using the TaqMan miRNA Low Density Array System (Applied Biosystems). The miRNA having the increased expression as compared with the control was screened from the sample after being treated.

As a result, 146 miRANs that were highly expressed after being treated with a drug were found in two cell lines, T24 and UM-UC-3, in common. Among these miRNAs, there were 23 kinds of miRNA having a CPG island existed in the region within the upper 5kb of the pre-miRNA (registered in the above-described miRBASE database). These genes are highly expressed in the case of inhibiting the methylation, and thus have the possibility for the expression to be suppressed by the methylation in the cell lines.

hsa-miR-10b, hsa-miR-124, hsa-miR-132, hsa-miR-137, hsa-miR-147b, hsa-miR-148a, hsa-miR-152, hsa-miR-185a, hsa-miR-193a-5p, hsa-miR-200b, hsa-miR-200b*, hsa-miR-203, hsa-miR-22, hsa-miR-330-5p, hsa-miR-34c-5p, hsa-miR-409-3p, hsa-miR-409-5p, hsa-miR-449b, hsa-miR-545*, hsa-miR-636, hsa-miR-639, hsa-miR-675, and hsa-miR-9.

A Methylation-specific PCR (MSP) method was performed for these miRNAs using T24, UM-UC-3, HT1197, HT-1376, and SW780, which are bladder cancer cell lines. As a result, the methylation was recognized for the following 12 miRNA genes.

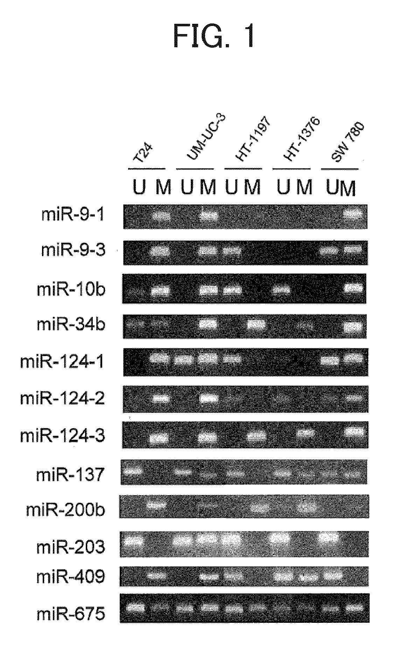

miR-34b, miR-9-1, miR-9-3, miR-124-1, miR-124-2, miR-124-3, miR-203, miR-10b, miR-675, miR-200b, miR-137, and miR-409. The results obtained from the MSP method are illustrated in Fig. 1. In the figure, U represents unmethylated DNA (Unmethylated) and M represents methylated DNA (Methylated).

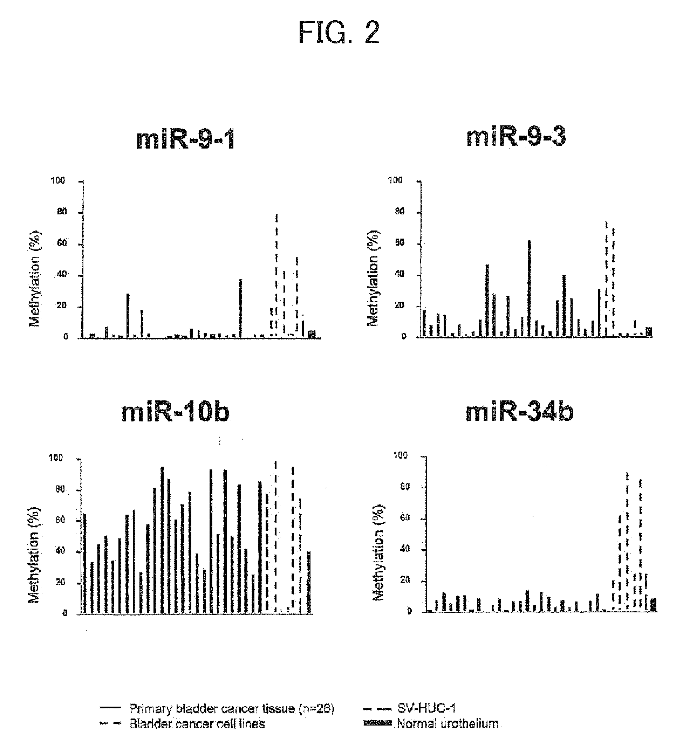

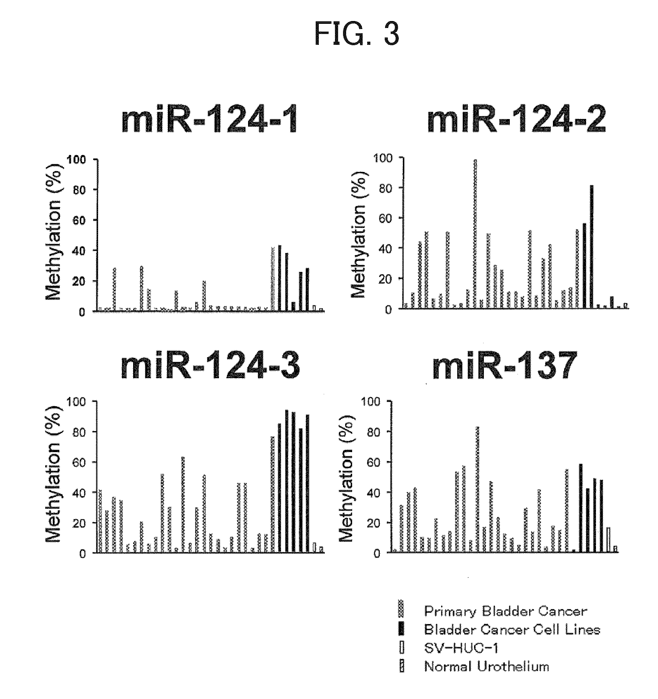

The results obtained by further analyzing the states of the methylation for these genes by a bisulfite pyrosequencing method are illustrated in Figs. 2, 3, and 4. In the figures, Methylation (%) of the vertical axis represents a degree of the methylation. Primary Bladder Cancer Tissue, Bladder Cancer Cell Lines, SV-HUC-1, and Normal Urothelium represent a result obtained by analyzing DNA derived from bladder cancer tissues removed by an operation, a result obtained by analyzing DNA derived from cell lines (T24, UM-UC3, HT1197, HT-1376, and SW780) of bladder cancer tissues, a result obtained by analyzing DNA derived from normal uroepithelial cell lines, and a result obtained by analyzing DNA (purchased from the company BioChain) derived from normal urothelium, respectively.

From these results, 12 kinds of miRNA that were methylated in bladder cancer tissues and bladder cancer cell lines were found.

### (Analysis of expression of miR-137 in bladder cancer cell lines)

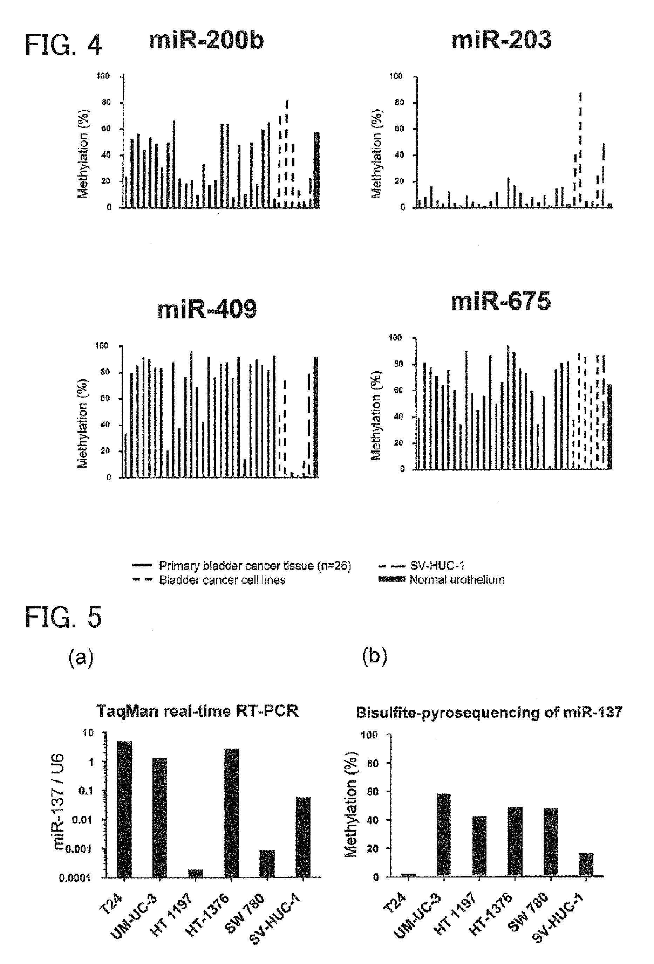

For miR-137 among the above-described miRNAs, the expression amounts of miR-137 in T24, UM-UC-3, HT1197, HT-1376, and SW780, which are bladder cancer cell lines, and SV-HUC-1, which is a normal uroepithelial cell line, were analyzed by a real time RT-PCR using TaqMan RT-PCR (the company Applied Biosystems), and the results thus obtained are illustrated in Fig. 5(a). These results demonstrate low expressions of miR-137 in HT1197 and SW780.

For these bladder cancer cell lines, the methylation of the miR-137 gene was analyzed by a bisulfite pyrosequencing method, and the results thus obtained are illustrated in Fig. 5(b). These results demonstrate the methylation of the miR-137 gene in UM-UC-3, HT1197, HT-1376, and SW780. The results in Figs. 5(a) and 5(b) demonstrate the possibility that the expressions of the miR-137 in the bladder cancer cell lines decrease, and the decrease occur due to the methylation.

### (Analysis of expressions of miRNAs for each cancer site)

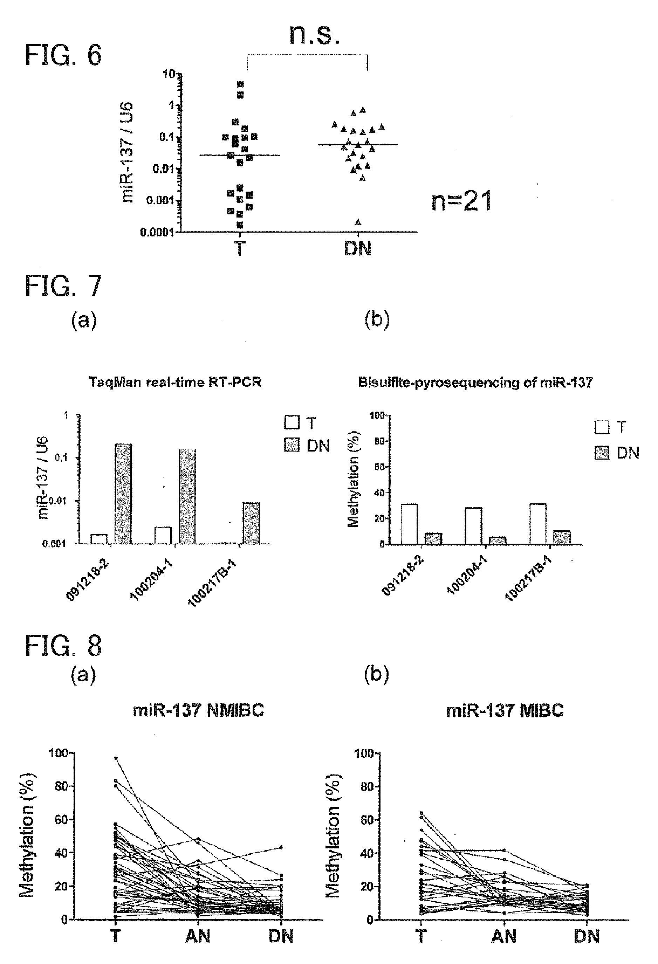

RNAs were extracted from cancer part tissues (T) and tissues that are considered to be normal tissues sufficiently apart from cancer and obtained at the time of removing cancer (DN; Distant Normal-appearing tissue), and then the expression amounts of miR-137 were analyzed by a real time RT-PCR. The results thus obtained are illustrated in Fig. 6. From these results, it is recognized that the expression amount tends to decrease in the cancer part (T). The difference that can be compared in the level of the expression amount is not recognized. However, it is considered that the reason is because it is difficult to detect the gene having a decreased expression amount due to the mixing of normal tissues for T.

Then, using T and DN that were some of the samples (091218-2, 100204-1, and 100217B-1) used in Fig. 6, the expression amount of miR-137 was analyzed by a real time RT-PCR, and the results thus obtained are illustrated in Fig. 7(a), and the methylation of miR-137 gene was analyzed by a bisulfite pyrosequencing method, and the results thus obtained are illustrated in Fig. 7(b). From these results, it is recognized that there is an inverse correlation between the expression amount of the miRNA and the methylation in the genome gene encoding the miRNA, in other words, the expression amount of the miRNA is decreased by the methylation of the CpG island that is a gene on the genome. The results have demonstrated that a decrease in the expression amount can be detected by analyzing the methylation of the miR-137 gene, in other words, it is possible to detect whether or not cancer cells exist.

For T collected from each of the cancer tissues, the tissue (AN; Adjacent Normal-appearing bladder tissue) that is 5 mm apart from the cancer part (collected tissue of T), and DN of (a) NMIBC (non-invasiveness and superficial property) and (b) MIBC (invasiveness), the methylation of the miR-137 gene was analyzed by a bisulfite pyrosequencing method, and the results thus obtained are illustrated in Fig. 8 (NMIBC (a) and MIBC (b)). It is to be noted that the results obtained from the same individual are connected in a line.

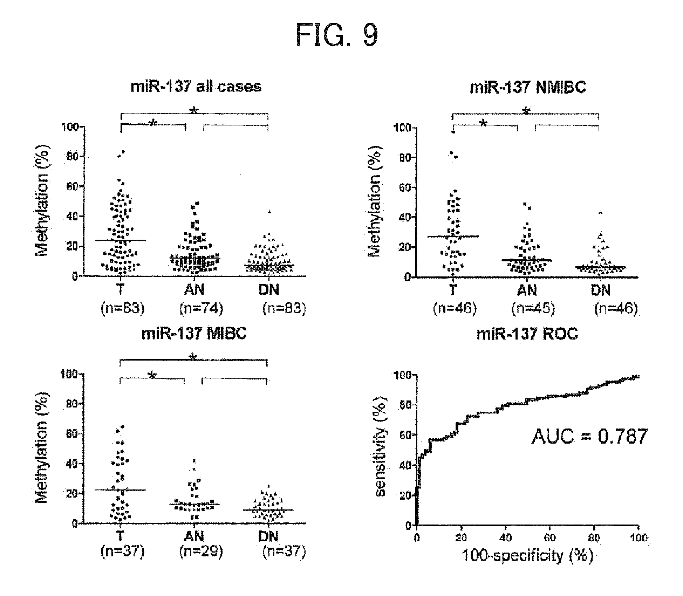

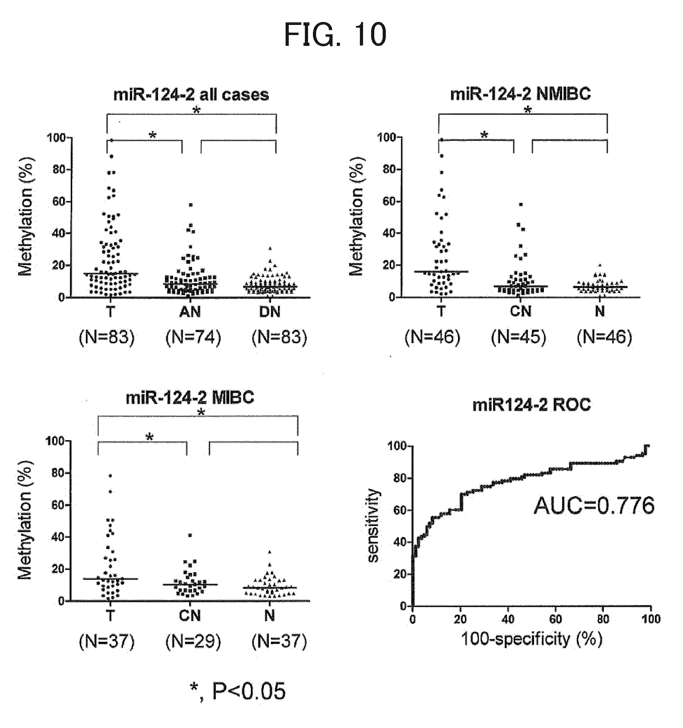

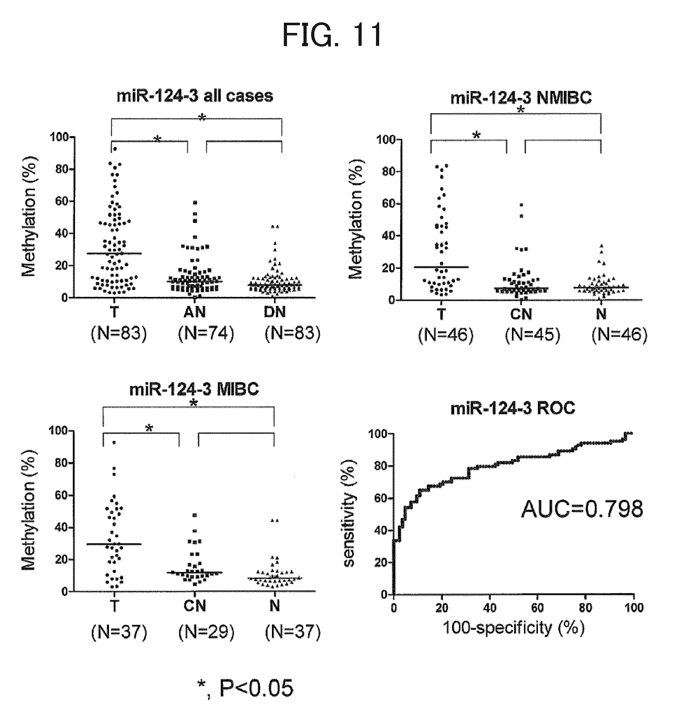

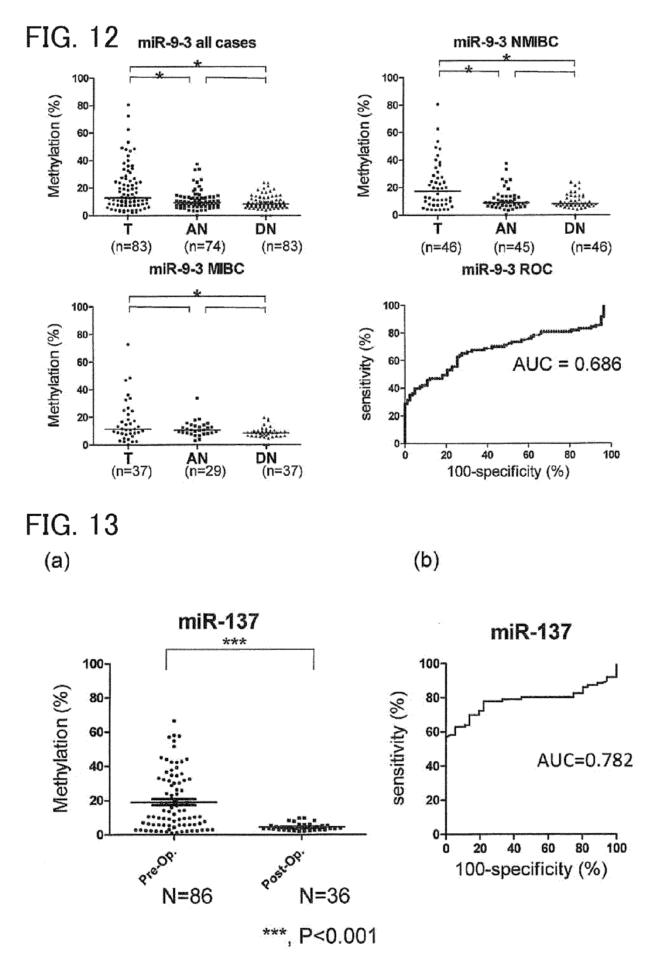

In addition, in a case of NMIBC, a case of MIBC, and both cases of NMIBC and MIBC of the miR-137 gene, the methylation was analyzed by a bisulfite pyrosequencing method and the ROC (receiver operating characteristic) curves of detection sensitivity (Sensitivity) and specificity (Specificity) were analyzed. The results thus obtained are illustrated in Fig. 9. The results obtained by performing the same analysis for the miR-124-2 gene (Fig. 10), the miR-124-3 gene (Fig. 11), and the miR-9-3 gene (Fig. 12) are also illustrated. There were significant differences between T and AN and T and DN in all of NMIBC and MIBC for miRNAs except for the miR-9-3 gene. It has been demonstrated that the methylation is decreased in the order of T, AN, and DN. There were no significant difference between T and AN of MIBC for the miR-9-3 gene, but there was the tendency that the methylation decreased. It is to be noted that the methylation for the miR-9-1 gene was analyzed (not illustrated), but the frequency of the methylation was very low. From these results, it is considered that the cancer part exhibits high methylation for these miRNAs as compared with the non-cancerous part, and the tendency of the methylation is helpful to specify the cancer site. In addition, it has been identified that there are differences in the frequency of the methylation even in the sequences of the genome genes encoding the same miRNA.

### (Analysis of the state of methylation of the miR-137 gene in a urine specimen)

For the urine specimens before an operation for the removal of cancer tissues (Pre-OP) and after the removal of cancer tissues (Post-OP) in a subject, the methylation of the miR-137 gene was analyzed by a bisulfite pyrosequencing method. The results thus obtained (a) and ROC curves (b) are illustrated in Figs. 13(a) and 13(b). It is noted that in the case of an abdominal operation for Post-OP, the whole bladder is removed and thus the urine specimen of the endoscope excision case is only obtained. Therefore, the number of N is small. There is the tendency that the methylation of the miR-137 gene after the operation decreases.

When ROC curves were made for the results of Fig. 13(a), and then the cut-off values were adjusted to maximize both sensitivity (Sensitivity) and specificity (Specificity), it was possible to detect with sensitivity, such as 78% of sensitivity, 78% of specificity, 0.89 of PPV, and 0.60 of NPV for 5.2% of a cut-off value.

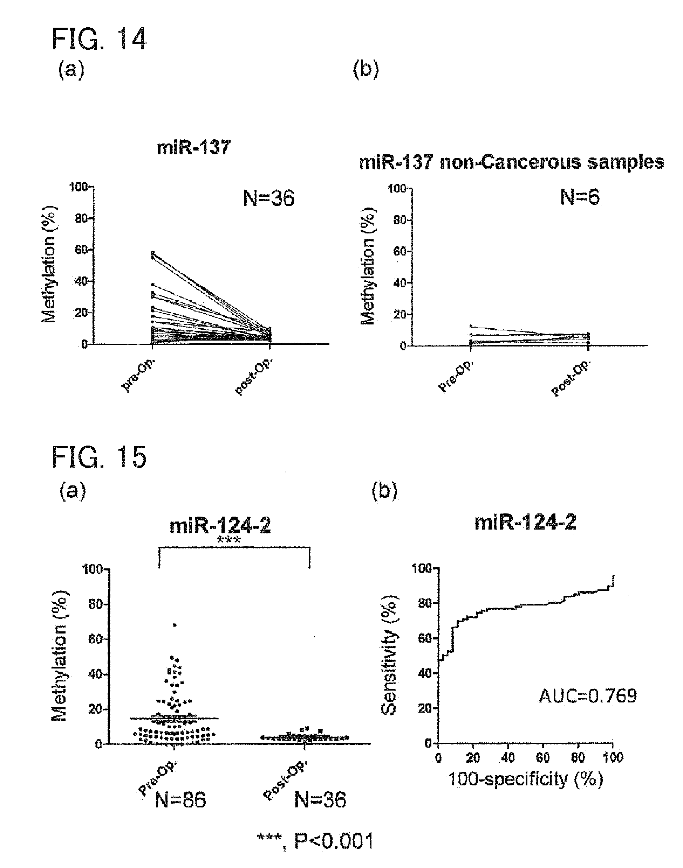

For (a) before and after an operation for the removal of cancer tissues and (b) before and after a removal operation of a non-cancerous subject (the case which was identified as having no cancer after the removal operation), the methylation of the miR-137 genes was analyzed by a bisulfite pyrosequencing method. The results thus obtained are illustrated in Figs. 14(a) and 14(b). It is to be noted that the results obtained from the same individual are connected in a line. The methylation of the miR-137 gene derived from the removed cancer tissues decreases; there are no changes in the methylation of the miR-137 gene derived from the non-cancerous tissues; and the above results are correlated with the results in urine specimens illustrated in Fig. 13(a). From these results, it is considered that the methylation of the miR-137 gene in a urine specimen is useful as a diagnostic marker of bladder cancer cells, and it has been demonstrated that the analysis of the methylation in urine can be applied for testing whether or not cancer tissues are present.

### (Analysis of state of methylation of miR-124-2 gene in urine specimen)

For the urine specimens before an operation for the removal of cancer tissues (Pre-OP) and after the removal of cancer tissues (Post-OP) of a subject, the methylation of the miR-124-2 gene was analyzed by a bisulfite pyrosequencing method. The results thus obtained (a) and the ROC curve (b) are illustrated in Figs. 15(a) and 15(b). There is the tendency that the methylation of the miR-124-2 gene after the operation decreases.

When ROC curves were made for the results of Fig. 15(a), and then cut-off values were adjusted to maximize both sensitivity and specificity, it was possible to detect with sensitivity, such as 70% of sensitivity, 89% of specificity, 0.94 of PPV, and 0.55 of NPV for 5.2% of a cut-off value.

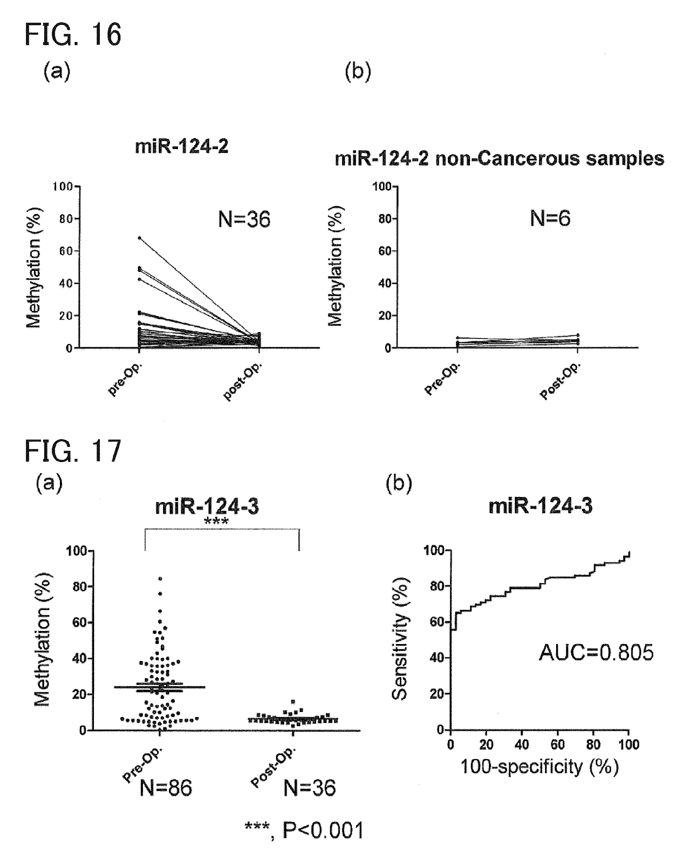

The results obtained (a) before and after an operation for the removal of cancer tissues and (b) before and after a removal operation of a non-cancerous subject are illustrated in Figs. 16(a) and 16(b). It is to be noted that the results obtained from the same individual are connected in a line. The methylation of the miR-124-2 gene derived from the removed cancer tissues decreases; there are no changes in the methylation of the miR-124-2 gene derived from the non-cancerous tissues; and the above results are correlated with the results in urine specimens illustrated in Fig. 15(a). From these results, it is considered that the methylation of the miR-124-2 gene in a urine specimen is useful as a diagnostic marker of bladder cancer, and it has been demonstrated that the analysis of the methylation in urine can be applied for testing whether or not cancer tissues are present.

### (Analysis of the state of methylation of miR-124-3 gene in urine specimen)

For the urine specimens before an operation for the removal of cancer tissues (Pre-OP) and after the removal of cancer tissues (Post-OP) of a subject, the methylation of the miR-124-3 gene was analyzed by a bisulfite pyrosequencing method. The results thus obtained (a) and the ROC curve (b) are illustrated in Figs. 17(a) and 17(b). There is the tendency that the methylation of the miR-124-3 gene after the operation decreases.

When ROC curves were made for the results of Fig. 17(a), and then cut-off values were adjusted to maximize both sensitivity and specificity, it was possible to detect with sensitivity, such as 65% of sensitivity, 97% of specificity, 0.98 of PPV, and 0.54 of NPV for 12% of a cut-off value.

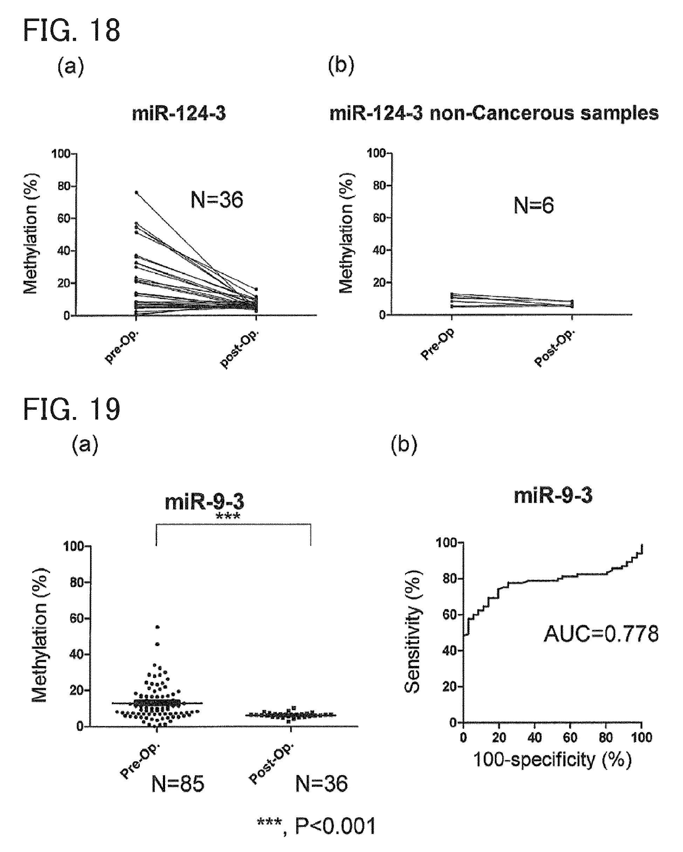

The results obtained (a) before and after an operation for the removal of cancer tissues and (b) before and after a removal operation of a non-cancerous subject are illustrated in Figs. 18(a) and (b). It is to be noted that the results obtained from the same individual are connected in a line. The methylation of the miR-124-3 gene derived from the removed cancer tissues decreases; there are no changes in the methylation of the miR-124-3 gene derived from the non-cancerous tissues; and the above results are correlated with the results in urine specimens illustrated in Fig. 17(a). From these results, it is considered that the methylation of the miR-124-3 gene in a urine specimen is useful as a diagnostic marker of bladder cancer, and it has been demonstrated that the analysis of the methylation in urine can be applied for testing whether or not cancer tissues are present.

### (Analysis of the state of methylation of miR-9-3 gene in urine specimen)

For the urine specimens before an operation for the removal of cancer tissues (Pre-OP) and after the removal of cancer tissues (Post-OP) of a subject, the methylation of the miR-9-3 gene was analyzed by a bisulfite pyrosequencing method. The results thus obtained (a) and the ROC curve (b) are illustrated in Figs. 19(a) and (b). There is the tendency that the methylation of the miR-9-3 gene after the operation decreases.

When the ROC curves were made for the results of Fig. 19(a), and then the cut-off values were adjusted to maximize both sensitivity and specificity, it was possible to detect with sensitivity, such as 69% of sensitivity, 86% of specificity, 0.92 of PPV, and 0.54 of NPV for 7.2% of a cut-off value.

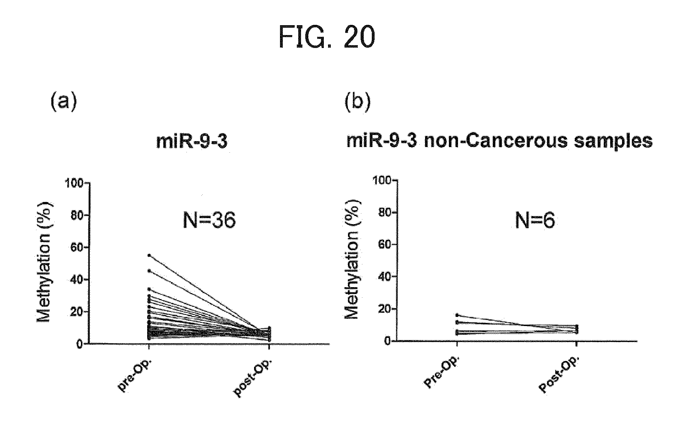

The results obtained (a) before and after an operation for the removal of cancer tissues and (b) before and after a removal operation of a non-cancerous subject are illustrated in Figs. 20(a) and 20(b). It is to be noted that the results obtained from the same individual are connected in a line. The methylation of the miR-9-3 gene derived from the removed cancer tissues decreases; there are no changes in the methylation of the miR-9-3 gene derived from the non-cancerous tissues; and the above results are correlated with the results in the urine specimens illustrated in Fig. 19(a). From these results, it is considered that the methylation of the miR-9-3 gene in a urine specimen is useful as a diagnostic marker of bladder cancer, and it has been demonstrated that the analysis of the methylation in urine can be applied for testing whether or not cancer tissues are present.

In addition, for four kinds of genes, such as the miR-137 gene, the miR-124-2 gene, the miR-124-3 gene, and the miR-9-3 gene, the technique for diagnosing bladder cancer using a urine specimen was examined and analyzed by combining the results of detecting each methylation.

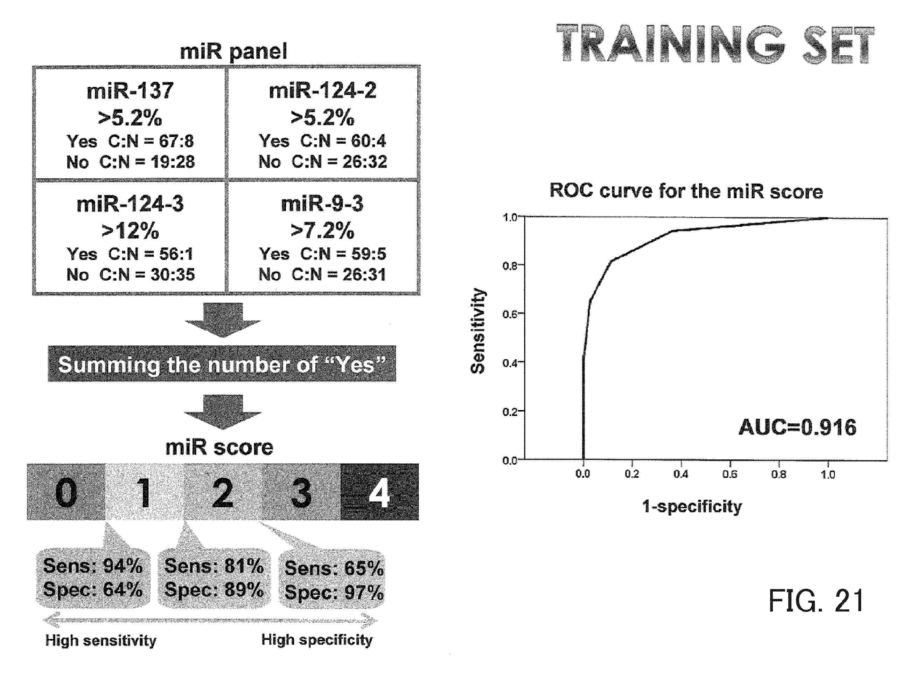

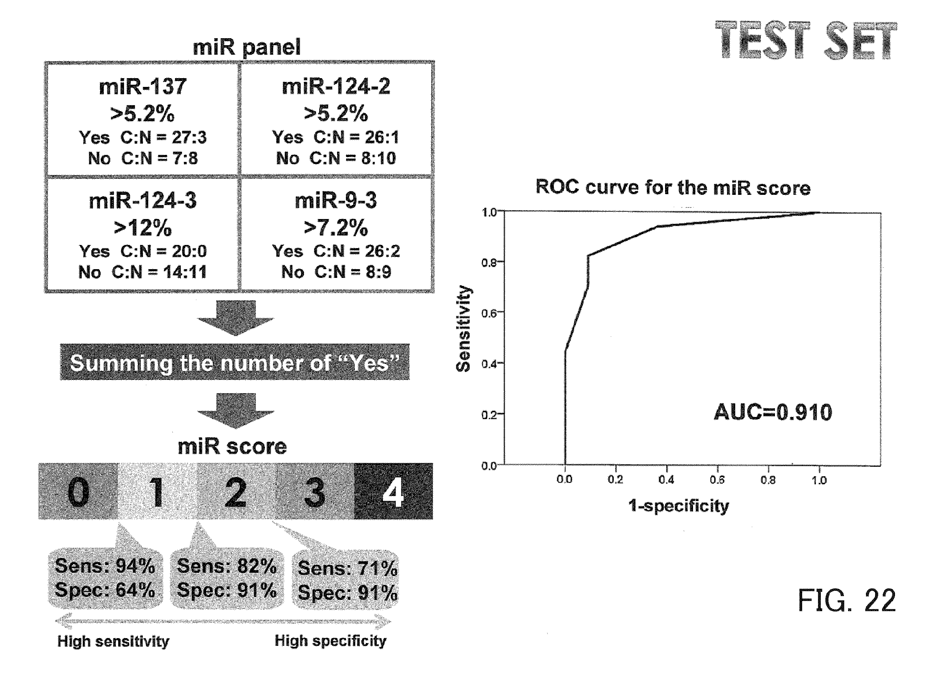

First, a panel detection method is illustrated in Figs. 21 and 22. In this method, the value, in which both the above-described sensitivity (Sens) and specificity (Spec) are maximized, is defined as a cut-off value, and is set to be 5.2%, 5.2%, 12%, and 7.2% for the miR-137 gene, miR-124-2 gene, miR-124-3 gene, and miR-9-3 gene, respectively. Then, when these values are satisfied, one point is added for each of the genes (Summing the number of "YES"). It was defined as miRscore, and then an ROC curve was made for each of the scores. As a result, between 0 and 1 point, the sensitivity was 94% and the specificity was 64%. Between 1 and 2 points, the sensitivity was 81% and the specificity was 89%. Between 2 to 3 points, the sensitivity was 65% and the specificity was 97%. In addition, these results were defined as a training set (Training Set) illustrated in Fig. 21, and then using a different new urine specimen the examination was again performed as a test set (Test Set) illustrated in Fig. 22. As a result, between 0 to 1 point, the sensitivity was 94% and the specificity was 64%. Between 1 and 2 points, the sensitivity was 82% and the specificity was 91%. Between 2 to 3 points, the sensitivity was 71% and the specificity was 91%. From these results, reproducibility for this detection method was confirmed.

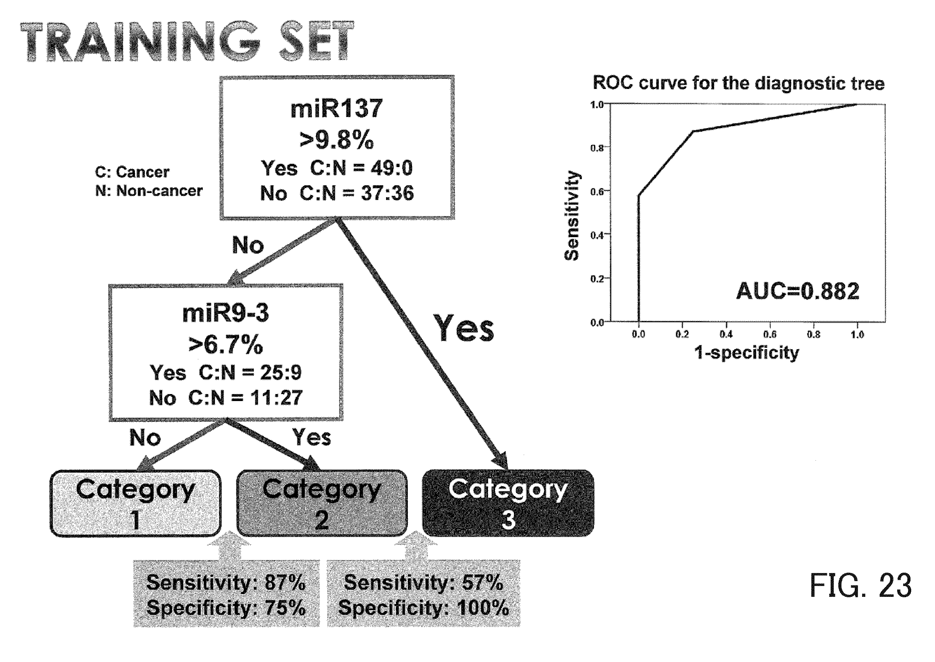

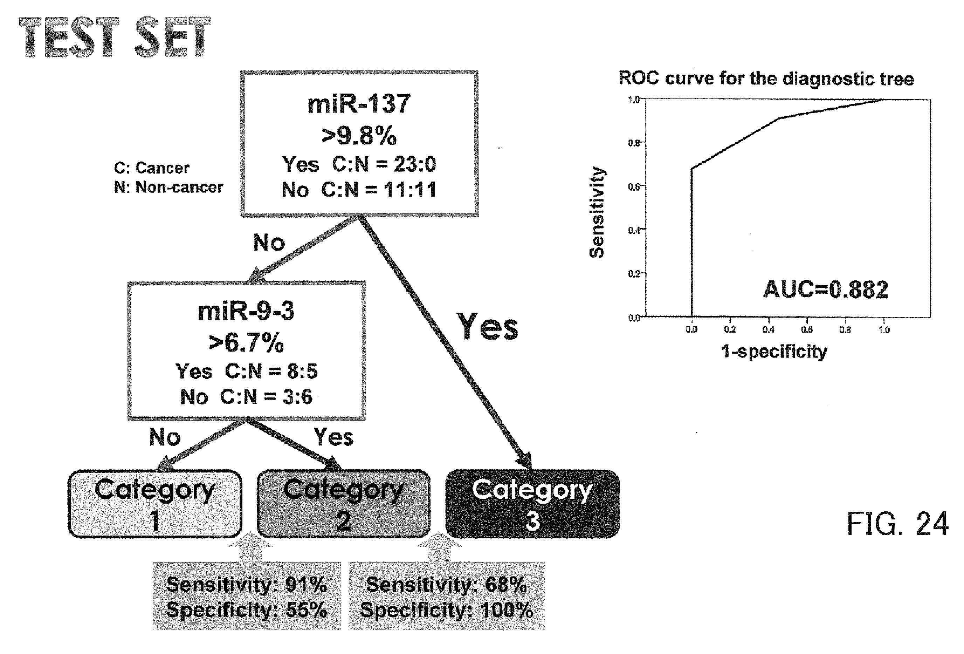

Next, the detection method by a Tree diagram is illustrated in Figs. 23 and 24. In this method, the methylation of the miR-137 gene that exhibits highest sensitivity at 100% of specificity among the four respective genes is divided by 9.8% of a cut-off value (57% of sensitivity). When this is satisfied, it is classified into Category 3. When this is not satisfied, the methylation of the miR-9-3 gene is further divided by 6.7% of a cut-off value. This value was the value that exhibited highest sensitivity and specificity when the ROC curve for each of the miR-124-2 gene, the miR-124-3 gene, and the miR-9-3 gene was prepared in a case in which the methylation of the miR-137 gene was 9.8% or less, and the sensitivity and specificity were 87% and 75%, respectively. When this is satisfied, it is classified into Category 2. When this is not satisfied, it is classified into Category 1. In addition, this was defined as the training set illustrated in Fig. 23, and using a different new urine specimen the examination was again performed as a test set illustrated in Fig. 24. As a result, Category 3 exhibited 100% specificity and 68% sensitivity and classifications of Category 1 and Category 2 exhibited 55% specificity and 91% sensitivity. From these results, reproducibility for this detection method was confirmed. It is to be noted that Category 3 exhibits 100% specificity, and when it is classified into Category 3, it is determined to be cancer with a high probability. When it is classified into Category 1, it is determined that the possibility of cancer is low, and when it is classified into Category 2, it is determined to be probably cancer.

The panel method and the Tree diagram method have demontrated the usefulness of the method for detecting cancer through combining the miR-137 gene, the miR-124-2 gene, the miR-124-3 gene, and the miR-9-3 gene. It is noted that those values are one example, and can be properly used by, for example, changing to the cut-off value indicating the sensitivity and specificity needed according to each scene such as screening, and postoperative follow-up.

The cut-off values of the respective genes that exhibit both maximum sensitivities and specificities in the urine specimens obtained from the above-described Examples (Figs. 13(a), 13(b), 15(a), 15(b), 17(a), 17(b), 19(a) and 19(b)), and the sensitivities and specificities in the case of using the corresponding cut-off values are listed in Table 1.

**[Table 1]**

| | miR-137 | miR-124-2 | miR-124-3 | miR-9-3 |
|---|---|---|---|---|
| Cut-off value | 5.2% | 5.2% | 12.0% | 7.2% |
| Sensitivity | 0.779 | 0.698 | 0.651 | 0.694 |
| Specificity | 0.778 | 0.889 | 0.972 | 0.861 |

In addition, the results of urinary cytodiagnosis in the above-described urine specimens are listed in Table 2.

**[Table 2]**

| Urinary cytodiagnosis | **Class I/II** | **Class III** | **Class IV/V** | **Total** |
|---|---|---|---|---|
| Number of patients (%) | 55(64%) | 15(17%) | 16(19%) | 86 |

As can be confirmed from Table 2, in the conventional urinary cytodiagnosis, only 19% of the positive reaction (Class IV, V) could be detected, and by combining 17% of the pseudo-positive reaction (Class III), only 36% could be detected for bladder cancer. In contrast to this, as listed in Table 1, any kind of the genes exhibit 0.65 or more of the value of sensitivity by analyzing the methylation even in the case of using the same urine specimen, and even in the case of using each of four kinds of the miRNAs alone, high sensitivity is obtained as compared with urinary cytodiagnosis.

### (Analysis of the suppressing effect of miRNA on bladder cancer)

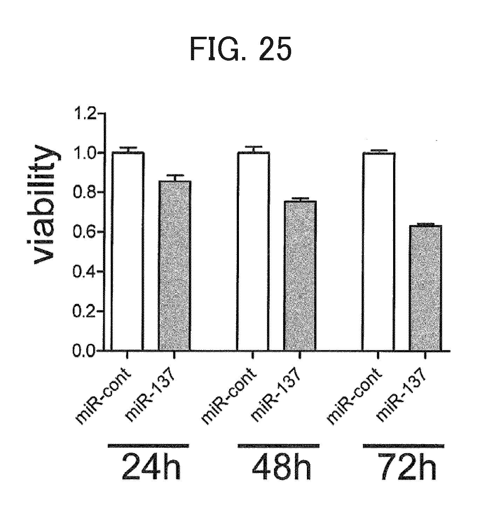

Since the expression of the miR-137 is decreased in bladder cancer, whether or not the miR-137 functions as a factor controlling cancer by forcibly expressing the miR-137 was analyzed. The miR-137 was transiently introduced into bladder cancer cells (SW780), and then forcibly expressed, and the results of comparing cell viabilities for miR-cont (expressing random sequences) are illustrated in Fig. 25. Viability in the figure represents cell viability. Since the expression amount of the miR-137 decreases for a long period of time due to the transient introduction, the cell viability was analyzed per 24 hours for 72 hours. The results were obtained in which the cell viability of cancer cells was decreased by the expression of the miR-137. The results have demonstrated the possibility that the miR-137 can be used as a bladder cancer inhibitor.

### (Analysis of detection precision of primary cancer)

The data of patients who exhibited pTa of an invasion depth and G1/G2 of an atypical degree, which are classified into primary cancer were only extracted from Table 1, and then the results thus extracted are listed in Table 3.

**[Table 3]**

| | miR-137 | miR-124-2 | miR-124-3 | miR-9-3 |
|---|---|---|---|---|
| Cut-off value | 5.2% | 5.2% | 12.0% | 7.2% |
| Sensitivity | 0.679 | 0.536 | 0.464 | 0.643 |
| Specificity | 0.778 | 0.889 | 0.972 | 0.861 |

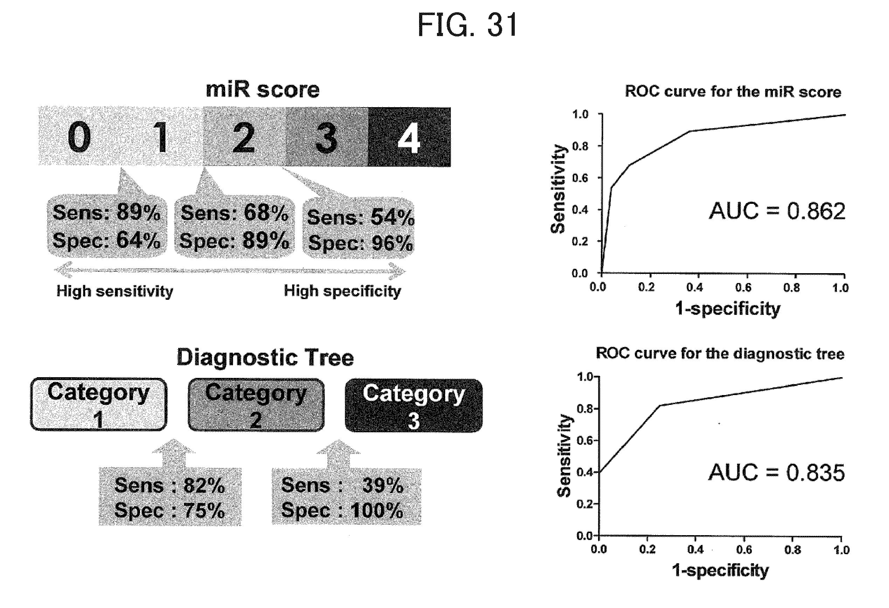

In addition, by combining the results in these genes, the combined results were analyzed by the panel detection method and the Tree diagram as illustrated in Figs. 21 to 24, and then ROC curves were prepared. This detection method is illustrated in Fig. 31. These results have revealed that the primary bladder cancer of a patient exhibiting pTa and G1/G2 can be detected by any of one or a combination of the respective genes through the analysis of the methylation of each of the genes.

In addition, the results of urinary cytodiagnosis in the urine specimens of the above-described patients with the primary cancer are listed in Table 4.

**[Table 4]**

| Urinary cytodiagnosis | **Class I/II** | **Class III** | **Class IV/V** | Total |
|---|---|---|---|---|
| Number of patients (%) | 24 (86%) | 4 (14%) | 0 (0%) | 28 |

As can be confirmed from Table 4, in the urinary cytodiagnosis, for the patients with the primary cancer exhibiting pTa and G1/G2, only 14% could be detected as the pseudo-positive reaction, but could not be detected as the positive reaction. In contrast to this, by analyzing the methylation of the above-described gene, the bladder cancer in low grade or low stage, which could not be detected in the urinary cytodiagnosis as the positive reaction, can be detected.

All of the above-described embodiments and Examples are intended to exemplify the invention, and are not intended to limit the present invention. The present invention can be performed by various modified embodiments and changed embodiments. Therefore, the scope of the present invention is defined by only the claims and an equivalent scope thereof.

### INDUSTRIAL APPLICABILITY

The present invention can be widely applied in medical and pharmaceutical fields for treating and preventing cancer.

## Claims

1. A method for detecting bladder cancer cells, the method comprising detecting an expression amount of a bladder cancer marker including one or more miRNAs selected from miR-124, miR-9, and miR-137 in a subject sample collected from a subject.

2. The method according to Claim 1, wherein the detection of the expression amount of the bladder cancer marker is to detect a decrease in the expression amount of the bladder cancer marker by detecting a methylation of a genome gene encoding the bladder cancer marker.

3. The method according to Claim 2, wherein the detection of the methylation is performed by a bisulfite pyrosequencing method.

4. The method according to any one of Claims 1 to 3, wherein the bladder cancer marker includes at least the miR-137.

5. The method according to any one of Claims 1 to 4, wherein the expression amount of the bladder cancer marker in the subject sample is compared with a threshold value.

6. The method according to any one of Claims 1 to 5, wherein the threshold value is an expression amount of the bladder cancer marker in a control sample collected from normal tissues.

7. The method according to any one of Claims 1 to 5, wherein the threshold value is an expression amount of the bladder cancer marker in a control sample collected from the subject at other times or collected from other tissues of the subject.

8. The method according to any one of Claims 1 to 7, wherein the subject sample is a urine sample.

9. A primer used in the method according to any one of Claims 1 to 8, wherein in the detection of the expression amount of the bladder cancer marker, the sequences of primers used for amplifying the miR-137 gene are a forward primer (GGGTTTAGYGAGTAGTAAGAGTTTTG) represented by SEQ ID NO 1 and a reverse primer (CCCCCTACCRCTAATACTCTCCTC) represented by SEQ ID NO 2.

10. The primer used in the method according to any one of Claims 1 to 8, wherein in the detection of the expression amount of the bladder cancer marker, the sequence of the primer used for the sequencing reaction of the miR-137 is GGTATTTTTGGGTGGATAAT represented by SEQ ID NO 3.

11. The primer used in the method according to any one of Claims 1 to 8, wherein in the detection of the expression amount of the bladder cancer marker, the sequences of primers used for amplifying the miR-124-2 are a forward primer (GTTGGGATTGTATAGAAGGATTATTTG) represented by SEQ ID NO 4 and a reverse primer (ACTACRAAAATCCAAAAAAAAATACATAC) represented by SEQ ID NO 5.

12. The primer used in the method according to any one of Claims 1 to 8, wherein in the detection of the expression amount of the bladder cancer marker, the sequence of the primer used for the sequencing reaction of the miR-124-2 is YGTTTTTATTGTTTTAGTTT represented by SEQ ID NO 6.

13. The primer used in the method according to any one of Claims 1 to 8, wherein in the detection of the expression amount of the bladder cancer marker, the sequences of primers used for amplifying the miR-124-3 are a forward primer (AAAAGAGAYGAGTTTTTATTTTTGAGTAT) represented by SEQ ID NO 7 and a reverse primer (TCCTCCRCAACTACCTTCCCCTA) represented by SEQ ID NO 8.

14. The primer used in the method according to any one of Claims 1 to 8, wherein in the detection of the expression amount of the bladder cancer marker, the sequence of the primer used for the sequencing reaction of the miR-124-3 is GAGATTYGTTTTTTTAAT represented by SEQ ID NO 9.

15. The primer used in the method according to any one of Claims 1 to 8, wherein in the detection of the expression amount of the bladder cancer marker, the sequences of primers used for amplifying the miR-9-3 are a forward primer (GATTTGAATGGGAGTTTGTGATTGGT) represented by SEQ ID NO 10 and a reverse primer (TCCCRAAACTCACRTAAAACACCC) represented by SEQ ID NO 11.

16. The primer used in the method according to any one of Claims 1 to 8, wherein in the detection of the expression amount of the bladder cancer marker, the sequence of the primer used for the sequencing reaction of the miR-9-3 is TTGGATTGAYGTTATTTT represented by SEQ ID NO 12.

17. The method according to Claim 2, wherein the detection of the methylation is performed by a methylation-specific PCR method.

18. A primer used in the method according to Claim 17, wherein in the methylation-specific PCR method, the sequences of primers used for detecting the miR-137 are a forward primer (GTAGCGGTAGTAGCGGTAGCGGT) represented by SEQ ID NO 13 and a reverse primer (GCTAATACTCTCCTCGACTACGCG) represented by SEQ ID NO 14 as allele-specific methylation primers and a forward primer (TGGTAGTGGTAGTAGTGGTAGTGGT) represented by SEQ ID NO 15 and a reverse primer (CCACTAATACTCTCCTCAACTACACA) represented by SEQ ID NO 16 as unmethylated allele-specific primers.

19. A primer used in the method according to Claim 17, wherein in the methylation-specific PCR method, the sequences of primers used for detecting the miR-124-2 are a forward primer (AGGGGCGTATTTTGGGGTTTTTGC) represented by SEQ ID NO 17 and a reverse primer (CCCCTACGACGTAATCGACCCG) represented by SEQ ID NO 18 as allele-specific methylation primers and a forward primer (TTTAGGGGTGTATTTTGGGGTTTTTGT) represented by SEQ ID NO 19 and a reverse primer (CATCCCCTACAACATAATCAACCCA) represented by SEQ ID NO 20 as unmethylated allele-specific primers.

20. A primer used in the method according to Claim 17, wherein in the methylation-specific PCR method, the sequences of primers used for detecting the miR-124-3 are a forward primer (GTTTTAGTGATAATCGGTCGGTGTC) represented by SEQ ID NO 21 and a reverse primer (TCCACGAAATCCACGCTACAAACG) represented by SEQ ID NO 22 as allele-specific methylation primers and a forward primer (TGTGTTTTAGTGATAATTGGTTGGTGTT) represented by SEQ ID NO 23 and a reverse primer (ATATCCACAAAATCCACACTACAAACA) represented by SEQ ID NO 24 as unmethylated allele-specific primers.

21. A primer used in the method according to Claim 17, wherein in the methylation-specific PCR method, the sequences of the primers used for detecting the miR-9-3 are a forward primer (GATTGACGTTATTTTTTCGCGGGGC) represented by SEQ ID NO 25 and a reverse primer (CGAAACTCACGTAAAACACCCGCG) represented by SEQ ID NO 26 as allele-specific methylation primers and a forward primer (TTGGATTGATGTTATTTTTTTGTGGGGT) represented by SEQ ID NO 27 and a reverse primer (CCCAAAACTCACATAAAACACCCACA) represented by SEQ ID NO 28 as unmethylated allele-specific primers.

22. A bladder cancer marker, containing one or more miRNAs selected from miR-124, miR-9, and miR-137.

23. The marker according to Claim 22, wherein the bladder cancer marker is used in a method for detecting bladder cancer cells, the method comprising detecting the expression amount of the bladder cancer marker in a subject sample collected from a subject.

24. The marker according to Claim 23, wherein the bladder cancer marker is used in the method for detecting bladder cancer cells in which the detection of the expression amount of the bladder cancer marker includes detecting the decrease in the expression amount of the bladder cancer marker by detecting the methylation of a genome gene encoding the bladder cancer marker.

25. The method according to Claim 2, wherein the detection is performed in a subject sample collected from a subject exhibiting pTa of an invasion depth or G1/G2 of an atypical degree.

26. A nucleic acid molecule having a nucleotide sequence represented by SEQ ID NO 1, SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24, SEQ ID NO 25, SEQ ID NO 26, SEQ ID NO 27, or SEQ ID NO 28.


SEQUENCE LISTING

<110>  Sapporo Medical University

<120>  Bladder cancer marker

<130>  -

<160>  43

<170>  PatentIn version 3.1

<210>  1
<211>  26
<212>  DNA
<213>  Artificial

<220>
<223>  primer for PCR amprification

<400>  1
gggtttagyg agtagtaaga gttttg                                          26


<210>  2
<211>  24
<212>  DNA
<213>  Artificial

<220>
<223>  primer for PCR amprification

<400>  2
ccccctaccr ctaatactct cctc                                            24


<210>  3
<211>  20
<212>  DNA
<213>  Artificial

<220>
<223>  primer for sequencing

<400>  3
ggtattttg ggtggataat                                                  20


<210>  4
<211>  27
<212>  DNA
<213>  Artificial

<220>
<223>  primer for PCR amprification

<400>  4
gttgggattg tatagaagga ttatttg                                         27


<210>  5
<211>  29
<212>  DNA

<213>  Artificial

<220>
<223>  primer for PCR amprification

<400>  5
actacraaaa tccaaaaaaa aatacatac                                          29


<210>  6
<211>  20
<212>  DNA
<213>  Artificial

<220>
<223>  primer for sequencing

<400>  6
ygtttttatt gttttagttt                                                    20


<210>  7
<211>  29
<212>  DNA
<213>  Artificial

<220>
<223>  primer for PCR amprification

<400>  7
aaaagagayg agtttttatt tttgagtat                                          29


<210>  8
<211>  23
<212>  DNA
<213>  Artificial

<220>
<223>  primer for PCR amprification

<400>  8
tcctccrcaa ctaccttccc cta                                                23


<210>  9
<211>  18
<212>  DNA
<213>  Artificial

<220>
<223>  primer for sequencing

<400>  9
gagattygtt tttttaat                                                      18


<210>  10
<211>  26
<212>  DNA
<213>  Artificial

<220>

<223>  primer for PCR amprification

<400>  10
gatttgaatg ggagtttgtg attggt                                          26


<210>  11
<211>  24
<212>  DNA
<213>  Artificial

<220>
<223>  primer for PCR amprification

<400>  11
tcccraaact cacrtaaaac accc                                            24


<210>  12
<211>  18
<212>  DNA
<213>  Artificial

<220>
<223>  primer for sequencing

<400>  12
ttggattgay gttatttt                                                   18


<210>  13
<211>  23
<212>  DNA
<213>  Artificial

<220>
<223>  Primer for MSP

<400>  13
gtagcggtag tagcggtagc ggt                                             23


<210>  14
<211>  24
<212>  DNA
<213>  Artificial

<220>
<223>  Primer for MSP

<400>  14
gctaatactc tcctcgacta cgcg                                            24


<210>  15
<211>  25
<212>  DNA
<213>  Artificial

<220>
<223>  Primer for MSP

<400>  15

tggtagtggt agtagtggta gtggt                                                25

<210>  16
<211>  26
<212>  DNA
<213>  Artificial

<220>
<223>  Primer for MSP

<400>  16
ccactaatac tctcctcaac tacaca                                               26

<210>  17
<211>  24
<212>  DNA
<213>  Artificial

<220>
<223>  Primer for MSP

<400>  17
aggggcgtat tttggggttt ttgc                                                 24

<210>  18
<211>  22
<212>  DNA
<213>  Artificial

<220>
<223>  Primer for MSP

<400>  18
cccctacgac gtaatcgacc cg                                                   22

<210>  19
<211>  27
<212>  DNA
<213>  Artificial

<220>
<223>  Primer for MSP

<400>  19
tttaggggtg tattttgggg tttttgt                                              27

<210>  20
<211>  25
<212>  DNA
<213>  Artificial

<220>
<223>  Primer for MSP

<400>  20
catcccctac aacataatca accca                                                25

<210>  21
<211>  25
<212>  DNA
<213>  Artificial

<220>
<223>  Primer for MSP

<400>  21
gttttagtga taatcggtcg gtgtc                                          25


<210>  22
<211>  24
<212>  DNA
<213>  Artificial

<220>
<223>  Primer for MSP

<400>  22
tccacgaaat ccacgctaca aacg                                           24


<210>  23
<211>  28
<212>  DNA
<213>  Artificial

<220>
<223>  Primer for MSP

<400>  23
tgtgttttag tgataattgg ttggtgtt                                       28


<210>  24
<211>  27
<212>  DNA
<213>  Artificial

<220>
<223>  Primer for MSP

<400>  24
atatccacaa aatccacact acaaaca                                        27


<210>  25
<211>  25
<212>  DNA
<213>  Artificial

<220>
<223>  Primer for MSP

<400>  25
gattgacgtt atttttcgc ggggc                                           25


<210>  26
<211>  24
<212>  DNA

<213>  Artificial

<220>
<223>  Primer for MSP

<400>  26
cgaaactcac gtaaaacacc cgcg                                              24


<210>  27
<211>  28
<212>  DNA
<213>  Artificial

<220>
<223>  Primer for MSP

<400>  27
ttggattgat gttatttttt tgtggggt                                          28


<210>  28
<211>  26
<212>  DNA
<213>  Artificial

<220>
<223>  Primer for MSP

<400>  28
cccaaaactc acataaaaca cccaca                                            26


<210>  29
<211>  23
<212>  RNA
<213>  Homo sapiens

<400>  29
uuauugcuua agaauacgcg uag                                               23


<210>  30
<211>  20
<212>  RNA
<213>  Homo sapiens

<400>  30
uaaggcacgc ggugaaugcc                                                   20


<210>  31
<211>  23
<212>  RNA
<213>  Homo sapiens

<400>  31
ucuuugguua ucuagcugua uga                                               23


<210>  32
<211>  102
<212>  DNA

<213>  Homo sapiens

<400>  32
ggtcctctga ctctcttcgg tgacgggtat tcttgggtgg ataatacgga ttacgttgtt    60

attgcttaag aatacgcgta gtcgaggaga gtaccagcgg ca                      102


<210>  33
<211>  109
<212>  DNA
<213>  Homo sapiens

<400>  33
atcaagatta gaggctctgc tctccgtgtt cacagcggac cttgatttaa tgtcatacaa    60

ttaaggcacg cggtgaatgc caagagcgga gcctacggct gcacttgaa               109


<210>  34
<211>  87
<212>  DNA
<213>  Homo sapiens

<400>  34
tgagggcccc tctgcgtgtt cacagcggac cttgatttaa tgtctataca attaaggcac    60

gcggtgaatg ccaagagagg cgcctcc                                        87


<210>  35
<211>  90
<212>  DNA
<213>  Homo sapiens

<400>  35
ggaggcccgt ttctctcttt ggttatctag ctgtatgagt gccacagagc cgtcataaag    60

ctagataacc gaaagtagaa atgattctca                                     90


<210>  36
<211>  595
<212>  DNA
<213>  Homo sapiens

<400>  36
ccacatcttc cctcctcact ggaaagacag cactcttctg tgttaagtat ttgattttgt    60

gatttgtctt tcagaattgg aaatagagcg gccatttgga tttgggcagg aagcagccga   120

gcacagcttt ggatccttct ttagggaaat cgagttatgg atttatggtc ccggtcaagc   180

tcagcccatc cccaggcagg ggcgggctca gcgagcagca agagttctgg tggcggcggc   240

ggcggcagta gcagcggcag cggtagcagc ggcagcggta gcagcggcag cggcagcttg   300

gtcctctgac tctcttcggt gacgggtatt cttgggtgga taatacggat tacgttgtta   360

ttgcttaaga atacgcgtag tcgaggagag taccagcggc aggggggcag cggccgccct   420

ccccagccca ccagctggcc actaaacgcc cgtggttgcc aaggtagcac tttcttgttc   480

ttttcatttc ctcgggtgtt ttcgcactgg ttccaccgga aaggctgtgc gctgcgcctc 540

tggtgaccag gactggaaaa tgggttcgcg cagggggcgg ggggcgaggt gaagg 595

<210> 37
<211> 595
<212> DNA
<213> Artificial (bisulfite)

<220>
<223> for bisulfite

<400> 37
ttatattttt tttttttatt ggaaagatag tatttttttg tgttaagtat ttgattttgt 60

gatttgtttt ttagaattgg aaatagagcg gttatttgga tttgggtagg aagtagtcga 120

gtatagtttt ggattttttt ttagggaaat cgagttatgg atttatggtt tcggttaagt 180

ttagtttatt tttaggtagg ggcgggttta gcgagtagta agagttttgg tggcggcggc 240

ggcggtagta gtagcggtag cggtagtagc ggtagcggta gtagcggtag cggtagtttg 300

gttttttgat ttttttcggt gacgggtatt tttgggtgga taatacggat tacgttgtta 360

ttgtttaaga atacgcgtag tcgaggagag tattagcggt aggggggtag cggtcgtttt 420

ttttagttta ttagttggtt attaaacgtt cgtggttgtt aaggtagtat ttttttgttt 480

tttttatttt ttcgggtgtt ttcgtattgg ttttatcgga aaggttgtgc gttgcgtttt 540

tggtgattag gattggaaaa tgggttcgcg tagggggcgg ggggcgaggt gaagg 595

<210> 38
<211> 340
<212> DNA
<213> Homo sapiens

<400> 38
cgatcgtttc gattctcttg ccggtcttgc agtttgcagg gtcctaaacg aagaggggtg 60

gagattcgag agtgcgggtc tggcgtgtgg tccaagggag ggggtgaggc tgggaaaggg 120

ggtgcagaat gcgggaacgg ctgggactgc acagaaggac catctgcgga ctcgttttca 180

ctgctccagc tcccgaaatc gtttctctgg acgcggcagt atccgcagcg catgcaccct 240

cctctggact tccgcagccc agacctgcgc ttacagtcat cgcgccaccc cctccccccg 300

ggctgcgggc cccgcgctcc gtgggctcag ggtcccctcc 340

<210> 39
<211> 340
<212> DNA
<213> Artificial (bisulfite)

<220>
<223> for bisulfite

<400> 39

cgatcgtttc gatttttttg tcggttttgt agtttgtagg gttttaaacg aagaggggtg 60

gagattcgag agtgcgggtt tggcgtgtgg tttaagggag ggggtgaggt tgggaaaggg 120

ggtgtagaat gcgggaacgg ttgggattgt atagaaggat tatttgcgga ttcgttttta 180

ttgttttagt tttcgaaatc gtttttttgg acgcggtagt attcgtagcg tatgtatttt 240

tttttggatt ttcgtagttt agatttgcgt ttatagttat cgcgttattt ttttttttcg 300

ggttgcgggt ttcgcgtttc gtgggtttag ggtttttttc 340

<210> 40
<211> 340
<212> DNA
<213> Homo sapiens

<400> 40
tctgggtctc cccgtctttc tctccacgaa cagctcgagc gccttctcgc gggcccgctg 60

cgcgcggaga ggacgagctc gctgggttgt aaaaagagac gagttttcat ctttgagcat 120

cgagattcgt tcttttaacc gcattcggtg cgcgctcctg ggtcggcacg ggcagggcga 180

cggcagggga aggcagctgc ggaggagctc gcgccgccca gtcggagcgg ttctgcgccc 240

ctcggagccc cgcgggaggc ggccgggtgc gcacgcgctc accaccccca cccccggaat 300

ccgtcttcgc gattcccggg cgccccagct ccaggaacgc 340

<210> 41
<211> 340
<212> DNA
<213> Artificial (bisulfite)

<220>
<223> for bisulfite

<400> 41
tttgggtttt ttcgtttttt tttttacgaa tagttcgagc gttttttcgc gggttcgttg 60

cgcgcggaga ggacgagttc gttgggttgt aaaaagagac gagtttttat ttttgagtat 120

cgagattcgt ttttttaatc gtattcggtg cgcgtttttg ggtcggtacg ggtagggcga 180

cggtagggga aggtagttgc ggaggagttc gcgtcgttta gtcggagcgg ttttgcgttt 240

ttcggagttt cgcgggaggc ggtcgggtgc gtacgcgttt attattttta ttttcggaat 300

tcgttttcgc gattttcggg cgttttagtt ttaggaacgt 340

<210> 42
<211> 425
<212> DNA
<213> Homo sapiens

<400> 42
tcagacggtg cgcagccctg aggcttctga gggcagaggg tgcgcttcct tgccctcggg 60

gcggggaagc cgtgaacgcc gaggccattg taaggctggg tggtgcggag cgcgggaagg 120

gggctgggat ttgaatggga gcctgtgatt ggccgatccc tggactgacg tcacttcccc 180

gcggggcgat tagcctgcga gaggagggcc ggcggtccag tgcgctgggg gcggccgggg 240

cgctcgaggc tctctaagcg ctccgcgcgg gtgccctacg tgagccccgg gacgccgtcg 300

aggcaggccg gcagcgccgg tgccaggacg cacggaacgg ggagcagggg agaaatgcgc 360

cgggagggcg agggaggaag ggaactgggc gggggctgcg ggcctaggtg gcgggagtca 420

gcgtg 425

<210> 43
<211> 425
<212> DNA
<213> Artificial (bisulfite)

<220>
<223> for bisulfite

<400> 43
ttagacggtg cgtagttttg aggtttttga gggtagaggg tgcgtttttt tgttttcggg 60

gcggggaagt cgtgaacgtc gaggttattg taaggttggg tggtgcggag cgcgggaagg 120

gggttgggat ttgaatggga gtttgtgatt ggtcgatttt tggattgacg ttattttttc 180

gcggggcgat tagtttgcga gaggagggtc ggcggtttag tgcgttgggg gcggtcgggg 240

cgttcgaggt tttttaagcg tttcgcgcgg gtgttttacg tgagtttcgg gacgtcgtcg 300

aggtaggtcg gtagcgtcgg tgttaggacg tacggaacgg ggagtagggg agaaatgcgt 360

cgggagggcg agggaggaag ggaattgggc gggggttgcg ggtttaggtg gcgggagtta 420

gcgtg 425